# EUROPEAN PATENT SPECIFICATION

(11) **EP 1 328 811 B1**
(45) Date of publication and mention of the grant of the patent: **31.08.2005**
(21) Application number: 01951273.0
(22) Date of filing: 06.07.2001
(51) Int. Cl.: G01N 33/576, C07K 14/18

(54) **HCV MOSAIC ANTIGEN COMPOSITION**
HCV MOSAIK ANTIGEN ZUSAMMENSETZUNG
COMPOSITION D'ANTIGENE MOSAIQUE DU VIRUS DE L'HEPATITE C (VHC)

(30) Priority: 07.07.2000 CA 2311022
(43) Date of publication of application: 23.07.2003
(73) Proprietor: MedMira Inc., Halifax, Nova Scotia B3S 1B3 (CA)
(72) Inventor: CHAN, Hermes, K.W., Halifax, Nova Scotia B3S 1H4 (CA); THEOLIS, Richard, Jr., Herring Cove, Nova Scotia B3V 1H2 (CA)
(74) Representative: Goddar, Heinz J.
(86) International application number: PCT/CA2001/000988
(87) International publication number: WO 2002/004484

(56) References cited:
- EP-A- 0 484 787
- EP-A- 0 489 968
- EP-A- 0 507 615
- JACKSON P ET AL.: "Reactivity of Synthetic Peptides Representing Selected Sections of Hepatitis C Virus Core and Envelope Proteins With a Panel of Hepatitis C Virus-Seropositive Human Plasma" JOURNAL OF MEDICAL VIROLOGY, vol. 51, no. 1, January 1997 (1997-01), pages 67-79, XP001053072

## Description

### FIELD OF INVENTION

The invention provides a highly immunoreactive mosaic antigen composition containing a plurality of different antigenic peptides encoded from the core region of the hepatitis C virus (HCV) genome for the purpose of detecting antibodies to HCV. An *in vitro* diagnostic method for detecting anti-HCV antibodies in a test sample and a diagnostic test kit are also provided which utilize the mosaic antigen composition as an immunoreagent.

### BACKGROUND OF INVENTION

Hepatitis C virus (HCV) is the principal cause of liver disease worldwide. Up to 85% of those infected with HCV become chronic carriers, which can lead to physiological disorders such as cirrhosis (scarring of the liver tissue), liver failure and cancer of the liver.

In 1998, the World Health Organization (WHO) estimated that 3% of the world population have been infected with HCV, which means there are more than 170 million HCV chronic carriers around the world.

Chronic HCV is the leading cause of liver transplants. It is also a major cause of cancer of the liver in many areas of the world. Chronic HCV often occurs without symptoms and most of those infected may remain unaware of their infection for years. It can take 20 years or more for symptoms to manifest in people who contract chronic liver disease. For this reason, HCV is called a silent epidemic.

HCV is transmitted primarily through infected blood. It may also be transmitted through sexual contact and vertically from infected mothers to their newborns.

HCV was discovered in the late 1970s as the etiologic agent of hepatitis C. HCV is a RNA virus belonging to the Flaviviridae family. However, it was not until 1989 that a test for specific antibodies to the virus became available, thus permitting routine testing.

No vaccine is presently available for HCV. In recent years, promising treatment options for hepatitis C have emerged. However, because chronic hepatitis C is often silent, it is usually discovered only by routine clinical testing.

A simple and quick HCV serologic test could improve screening and diagnostic services, and is an appealing alternative in many settings that may have limited resources and facilities. According to the U.S. Center for Disease Control and Prevention report, rapid or point-of-care tests also enable healthcare providers to supply within minutes the test results to patients at the time of testing, thus potentially increasing the overall effectiveness of counseling and screening programs. Since prevention is an important aspect of pandemic control, reliable access to adequate diagnostic methods is an essential requirement for public health protection. However, traditional methods presently available for diagnosing HCV infection have several disadvantages.

There are essentially three well-defined strategies that are currently utilized in the diagnosis of HCV infection which are based on whether the target analyte is: (a) viral nucleic acid, (b) viral antigens, or (c) an antibody specific to the viral antigens. Depending upon the particular circumstances and the type of Information sought, an assay used for the purpose of detecting HCV infection may incorporate any one of these three methods, or a combination thereof.

Although positive detection of viral nucleic acid is the foremost indicator of the presence of the virus, there are certain disadvantages which are attributed to using this type of approach. For example, molecular assays allowing detection of viral nucleic acid (RNA) are, in general, significantly more complex and require more time than most assays for detection of viral antigens and anti-HCV antibodies. In general, nucleic acid detection assays involve complex procedures such as reverse transcriptase polymerase chain reaction (RT-PCR) techniques, nucleic acid fragment separation by electrophoresis and Northern blot analyses. Therefore, the complexity of this approach associated with the requirement for sophisticated laboratory equipment and a high level of technical skill and expertise compromises its application in a clinical setting. Furthermore, this type of diagnostic assay will only be effective in obtaining an accurate result if the level or concentration of viral nucleic acid in a sample is sufficiently high enough to be adequately detected. As such, the application of this particular method is often restricted to a biological test sample of an appropriate tissue (e.g. a liver biopsy) in which viral nucleic acid is likely to be present at a very high level, thus imposing certain limitations on the types of biological samples that are suitable for providing an accurate diagnosis.

When determining viral antigens as the target analyte, the immunological assay is generally designed such that one or more monoclonal or polyclonal antibodies specific to the viral antigens are used as detection and/or capture reagents. The most common format of this assay is the immunometric format, also called the double determinant format or the double antibody sandwich format. In this type of assay, the viral antigen (or bacterial antigen) to be detected is usually brought into contact with a specific monoclonal antibody that is often immobilized to a solid support (i.e. capture antibody). Following incubation with a biological sample suspected of containing the antigen, the immobilized monoclonal antibody selectively binds the antigen. After a washing step, the sample is then incubated with a labeled antibody (i.e. probe or detection antibody) resulting in the antigen being trapped between the capture antibody and the probe antibody. However, one known disadvantage with using this method is encountered when endeavoring to generate antibodies that can recognize the antigen with high affinity and specificity. In addition, the antibodies must possess the ability to recognize equally the all naturally occurring variant forms of the antigen. This aspect is particularly relevant in the case of viral and bacterial antigens which show a high degree of sequence diversity among the different strains.

An alternative approach to diagnosing infectious disease is to detect the antibody specific to the infectious agents. In most infectious (bacterial and viral) diseases, significant antibody response is produced by the host immune system. Since the presence of antibodies specific to an infectious agent is indicative of an on-going infection, the detection of specific antibodies is a very attractive approach in the diagnosis of infectious disease states. In this regard, targeting antibodies provides a more practical and reliable means of detecting viral infectious diseases compared to antigens since in many instances, antigens are not normally found at adequate concentrations for detection in biological fluids (e.g. blood and urine).

The most commonly used test format for antibody detection is the antibody capture assay format. According to this format, the antigen(s) corresponding to the infectious agent are immobilized onto a solid phase and placed in contact with a biological sample suspected of containing antibodies specific to the infectious agents. Following a suitable incubation time which allows an immune complex (i.e. antibody-antigen complex) to form, the antibodies specific to the infectious agents bind to the immobilized antigen(s) (i.e. capture reagent). Subsequent to the incubation phase, the immune complexes can be detected using standard immunochemical methods. One major advantage provided by this test format is that in most infectious disease states, high antibody titers can be found in blood circulation and consequently permits the development of highly sensitive methods for infectious disease diagnosis. Accordingly, serological screening and diagnosis of hepatitis C is routinely undertaken by the detection of antibodies against HCV in serum or plasma.

The most commonly used assay format for detecting anti-HCV is the ELISA (enzyme-linked immunosorbent assay). A progressive improvement in the sensitivity of anti-HCV antibody tests has been accomplished through three generations of ELISA screening assays by incorporating different configurations or types of capture immunoreagents (antigens) such as recombinant viral proteins or mixtures of synthetic polypeptides corresponding to various regions of the HCV polyprotein.

Recombinant proteins or synthetic peptides employed as capture immunoreagents for the detection of HCV antibodies contain sequences derived from highly conserved regions of the nonstructural proteins of the virus (e.g. NS3, NS4 and NS5), and the structural proteins (core and envelope proteins). The selection of the recombinant antigen and synthetic peptide antigen sequences is based on the location of the major antigenic determinants (epitopes) of the HCV polyprotein. Whether an assay employs recombinant protein antigens or synthetic peptide antigens, both forms of antigens must contain most antigenic determinants of the HCV polyprotein in order to exhibit the specificity and sensitivity required to achieve suitable performance for HCV diagnosis. Typically, immunoassays designed to detect antibodies specific to the HCV polyprotein use either a combination of synthetic peptides as capture immunoreagents, or one or more recombinant antigen proteins which may contain all major antigenic sites in one long contiguous polypeptide chain. This is because usually one synthetic peptide contains only one antigenic determinant and in general, there is more than one antigenic determinant for a protein molecule, e.g. HCV polyprotein.

For instance, EP 0 489 968 A1 discloses the use of combinations of HCV-derived peptides to detect HCV antibodies in sera, using an ELISA immunoassay. A similar approach is also used by Jackson et al. (J. Med. Virol. [1997] 51:67-79), in which homogeneous samples of HCV peptides coupled to bovine serum albumin (BSA) are used to detect HCV antibodies in serum. However, these assays have relatively poor sensitivity and selectivity, and require significant amounts of time and expertise to arrive at the desired result.

Many drawbacks are associated with the use of recombinant antigen proteins. Firstly, the three-dimensional conformation of the polypeptide chain resulting from aberrant folding can result in sequestration of epitopes from the surface of the protein. As such, the effectiveness in providing a reasonable degree of binding to an antibody may be reduced or abolished, thereby resulting in compromised sensitivity and specificity as an immunoreagent. In addition, aberrant folding of the polypeptide chain may produce structural epitopes not present in the native antigens which are likely to promote cross-reactivities and non-specific binding with other antibodies, thereby potentially yielding false positive reactions. Secondly, other disadvantages that are commonly encountered during upscale production of recombinant proteins and which represent serious limitations regarding their use are: low level expression of the recombinant antigen; instability of the expression system; degradation due to proteolysis; poor solubility of the recombinant antigens in aqueous solutions due to a propensity to aggregate; and low recovery during processing and purification steps. Accordingly, the development of highly specific and reliable diagnostic tests for HCV infection has been problematic using presently available methods.

Thus, while current methods have proven useful for the determination of HCV infection, there remains a need for a diagnostic assay which incorporates the use of an immunoreagent that avoids the apparent disadvantages described above and at the same time, provides a highly sensitive and specific analytical test.

According to the present invention, there is provided a HCV mosaic antigen composition which, when utilized as an immunoreagent in a HCV screening assay, provides a highly sensitive and accurate test for the *in vitro* detection of antibodies to HCV in human serum, plasma or whole blood and which comprises two or more different peptides, characterized in that each peptide: consists of an amino acid sequence selected from the group consisting of MSTNPKPQRKTKRNTNRR (SEQ ID NO:1), KTKRNTNRRPQDVKF (SEQ ID NO:2), GQIVGGVYLLPRRGP (SEQ ID NO:3), GPRLGVRATRKTSERSQP (SEQ ID NO:4), ATRKTSERSQPRGRRQPI (SEQ ID NO:5), and PKARQPEGRAWAQPG (SEQ ID NO:6); is covalently coupled N-terminally or C-terminally to a carrier component; and has a peptide to carrier mass ratio in the range of 0.5 to 8.0. The mosaic antigen composition is intended for use in clinical diagnostic testing whereby blood donors or other individuals are screened to determine whether or not they are HCV infected.

### SUMMARY OF THE INVENTION

Recombinant fusion proteins and long-chain polypeptides comprising a portion or entire regions of the HCV genome possess certain disadvantages related to their development and utilization in diagnostic assays. In spite of major technical advances made in the field of recombinant expression and purification, their application as immunoreagents for diagnostic testing still suffer from major drawbacks due to the technical issues mentioned above. As outlined previously, recombinant fusion proteins are plagued with problems associated with complicated processing and purification methods, their susceptibility to proteolysis and degradation, poor solubility in aqueous solutions and the potential for generating false positive reactivities; the latter resulting from the formation of cross-reactive epitope sites based upon structural homologies between the fusion protein and unrelated antigens. Furthermore, because recombinant fusion proteins are difficult to purify and process, there is a potential risk of contamination by foreign proteins derived from the host cells used in the expression system.

Producing peptides through chemical synthesis using automated methods overcomes some of the problems identified with using DNA recombinant means. However, when the length of the peptide is greater than about forty or fifty amino acid residues in length, it becomes increasingly difficult to produce a product of high Integrity with consistent yields. Accordingly, chemical synthesis and purification of relatively long-chain polypeptides can often lead to substantial sequence error that can seriously compromise diagnostic performance. In addition, increased peptide length is associated with a poor production yield, high production costs, and physical characteristics which may promote aberrant folding thereby leading to poor solubility.

Designing and producing synthetic peptides with much shorter amino acid sequences overcomes the disadvantages described above for long-chain polypeptides and recombinant fusion proteins, but depending on the size of the molecule, may not be sufficiently immunoreactive for diagnostic purposes. This is because although the sequence may contain an epitopic site, a short-chain peptide may not be oriented in the proper configuration necessary to fully express the epitope structural motif, thereby allowing optimal antibody recognition. However, by coupling the antigenic peptides individually through orientation specific covalent linkage to a carrier component, each antigenic determinant can be fully exposed for recognition and binding to an anti-HCV antibody. Furthermore, by employing a combination of different overlapping peptides encoded from different regions of the HCV genome which correspond to the major antigenic determinants, a broader range of immunochemical reactivity may be achieved compared to a fusion protein or long-chain polypeptide. The resulting product is a mosaic antigen composition comprising a large number of epitopes that are associated only with the peptides of interest. By contrast, fusion proteins or long-chain polypeptides may contain only a limited number of epitopes, or poorly exposed epitopes, due to steric hindrance effects resulting from aberrant protein folding.

Therefore, an object of the present invention is to provide a mosaic antigen composition which is highly immunoreactive with anti-HCV antibodies and comprises two or more different peptides, characterized in that each peptide: consists of an amino acid sequence selected from the group consisting of MSTNPKPQRKTKRNTNRR (SEQ ID NO:1), KTKRNTNRRPQDVKF (SEQ ID NO:2), GQIVGGVYLLPRRGP (SEQ ID NO:3), GPRLGVRATRKTSERSQP (SEQ ID NO:4), ATRKTSERSQPRGRRQPI (SEQ ID NO:5), and PKARQPEGRAWAQPG (SEQ ID NO:6); is covalently coupled N-terminally or C-terminally to a carrier component; and has a peptide to carrier mass ratio in the range of 0.5 to 8.0 thereby avoiding the disadvantages of using recombinant fusion proteins and long-chain synthetic peptides.

The composition of the invention can be used for the detection and diagnosis of HCV infection due to its high specificity, selectivity and sensitivity of binding to anti-HCV antibodies.

Still another object of the present invention is to provide a specific combination of different antigenic peptides of the invention derived from the core region of the HCV genome which encompass major antigenic sites and demonstrates a high level of immunoreactivity advantageous in the detection of anti-HCV antibodies.

### I. Immunoreactive Peptides

According to one aspect of the present invention, there is provided a mosaic antigen composition essentially comprising a plurality of different peptide fragments, preferably six in number, derived from the core region of the HCV genome which are covalently linked to a carrier component and which comprises two or more different peptides, characterized in that each peptide: consists of an amino acid sequence selected from the group consisting of MSTNPKPQRKTKRNTNRR (SEQ ID NO:1), KTKRNTNRRPQDVKF (SEQ ID NO:2), GQIVGGVYLLPRRGP (SEQ ID NO:3), GPRLGVRATRKTSERSQP (SEQ ID NO:4), ATRKTSERSQPRGRRQPI (SEQ ID NO:5), and PKARQPEGRAWAQPG (SEQ ID NO:6); is covalently coupled N-terminally or C-terminally to a carrier component; and has a peptide to carrier mass ratio in the range of 0.5 to 8.0. It has been determined that several epitopic sites are contained within the N-terminal region of the HCV core protein which are highly immunoreactive and therefore, useful in the detection of HCV antibodies. Thus, in accordance with the present invention, extensive serological analysis has led to the refinement and further definition of immunoreactive peptides that are useful in the detection and diagnosis of HCV infection. Furthermore, employing a unique combination of six different overlapping HCV peptides provides a mosaic antigen composition having a broader range of immunological reactivity and possesses a more consistent epitopic expression than HCV antigens produced by recombinant technology. Therefore, combinations of HCV antigen peptides identified and disclosed herein have increased sensitivity and greater specificity in providing more efficient and consistent detection of HCV antibodies. To determine the efficacy of the subject peptides in detecting HCV infection, individual peptides, as well as various combinations, were tested for their immunoreactivity with special specimens previously selected through the screening of patients with HCV and normal sera. Such serum panels have been well characterized and are provided in the Examples.

According to one particular aspect of the invention, there is provided six different HCV peptides having amino acid sequences defined as follows, which are used in the composition of the invention :

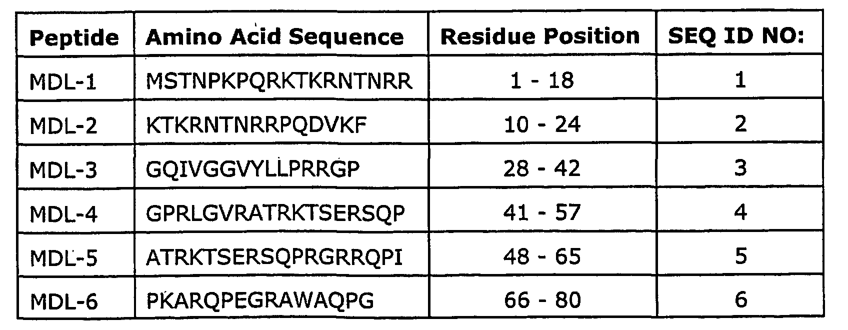

wherein the single and three letter codes for each amino acid residue is identified as:

| | | | | | |
|---|---|---|---|---|---|
| A | Ala | alanine | L | Leu | leucine |
| R | Arg | arginine | K | Lys | lysine |
| N | Asn | asparagine | M | Met | methionine |
| D | Asp | aspartic acid | F | Phe | phenylalanine |
| C | Cys | cysteine | P | Pro | proline |
| E | Glu | glutamic acid | S | Ser | serine |
| Q | Gln | glutamine | T | Thr | threonine |
| G | Gly | glycine | W | Trp | tryptophan |
| H | His | histidine | Y | Tyr | tyrosine |
| I | Ile | isoleucine | V | Val | valine |

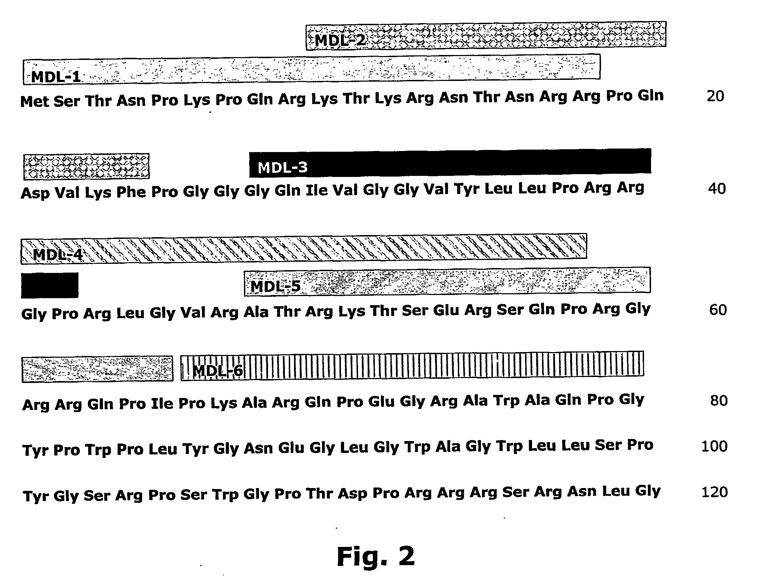

Figure 2 illustrates how these sequences span the amino acid residue positions of the HCV polyprotein encoded by the core region of the HCV genome.

The peptides defined by SEQ ID NO: 1, 2, 3, 4, 5 and 6 have been found to be exceptionally immunoreactive with HCV antibodies. An advantage of this reactivity is that the use of a combination of the peptides according to the invention will increase the specificity and the sensitivity of the immunological assay when compared to the use of recombinant fusion proteins or large polypeptides which only contain a limited number of antigenic determinants. Furthermore, it has been determined by the inventor through extensive experimentation, that the combination and selection of the six peptides described above, each of which spans a region of the core protein from positions 1 to 80, are optimal in providing increased sensitivity and specificity in detecting anti-HCV antibodies. A panel of overlapping peptides between positions 1 and 186 was synthesized and tested for immunoreactivity with a commercially available human serum panel. However, based on the experimental results, it was determined that all of the immunoreactivity was concentrated within the region between positions 1 to 80. Accordingly, employing any peptide derived from the core protein beyond position 80 would result in a composition exhibiting lower specificity and sensitivity and is therefore, less preferred in the practice of the invention.

According to the present invention, it has also been determined through extensive experimentation that the peptides range in length from 15 to 19 amino acid residues in order to function optimally as capture antigen. Following conjugation to a carrier component, there is an increased tendency for peptides having a length greater than 20 amino acid residues to fold or interact with the carrier component which can alter the expression of linear epitopes that are essential for HCV antibody recognition. On the other hand, it is expected that selecting peptides less than 12 amino acid residues in length will likely compromise their immunoreactivity due to the limited ability of an antibody to effectively recognize and bind to an antigenic determinant having less than a required minimal number of amino acid residues.

The peptides of the present invention can be prepared by methods known in the art, including chemical synthetic methods. Preferably, the peptides can be readily synthesized using standard techniques, such as the Merrifield method of synthesis [Merrifield (1963) J. Am. Chem. Soc. 85:2149-2154]. The advantages of using synthetic peptides are well known. For example, when synthetically prepared, the quality of the peptide compositions of the present invention can be controlled and as a result, reproducibility of the test results can be assured. Also, since very small amounts of a peptide are required for each test procedure, and because the expense of preparing a peptide is low, the resultant cost of screening body fluids for antibodies to HCV is relatively low. Another problem which can be minimized by using synthetic peptides is the avoidance of cross-reactivity with other antibodies and an increase in false positive results caused by the presence of antigenic materials from expression systems used in recombinant-based diagnostic tests.

It is expected that as long as the immunochemical activity of the peptides is preserved so that they remain recognizable by anti-HCV antibodies, several amino acids in the peptides according to the invention can be deleted, inserted or substituted by other amino acids or amino acid analogues or derivatives due to strain-to-strain variations among different isolates of HCV. The use of HCV antigenic peptides having such differing amino acid sequences is intended to be within the scope of the present invention, provided, however, the variation does not significantly diminish the immunochemical reactivity of the peptide with sera from persons infected with HCV. Other types of analogues or derivatives may also include chemical modifications of the peptide for facilitating conjugation to a carrier molecule.

According to another aspect of the present invention, there is provided a particular composition of different peptides selected from the group of amino acid sequences defined by SEQ ID NO: 1, 2, 3, 4, 5 and 6. The use of a peptide composition of the subject peptides provides the mosaic antigen composition with a broader spectrum of immunoreactivity against a greater number of HCV strains, thereby enhancing the ability of the antigenic peptides to react with antibodies specific for a wider range of HCV variants.

The various combinations of the subject peptides have been tested for their immunoreactivity in detecting anti-HCV antibodies through extensive experimentation, the results of which are provided in the Examples.

### II. Mosaic Antigen Composition

The mosaic antigen composition comprises a plurality of six different antigenic peptides derived from the core region of the HCV genome that are individually conjugated to a carrier component (e.g. proteins, synthetic polypeptides or polymers) or a chemically-active solid support surface. Preferably, the peptides are chemically coupled to a carrier component. More preferably, a protein is used which does not exhibit cross-reactivity with other antibodies in human serum and therefore, adversely affect the overall sensitivity and specificity of the assay.

The mosaic antigen composition of the present invention is formed by chemically coupling a plurality of small synthetic peptides to a carrier protein prior to its use as an immunoreagent. The antigenic peptides, defined by SEQ ID NO: 1, 2, 3, 4, 5 and 6, have been made sufficiently antigenic for recognition and binding by an antibody by conjugating either the amino or carboxyl terminal end of each individual peptide to a carrier protein. Based on the particular selection and concentration of peptides of the invention which are assembled together in the form of a conjugate, the summation of antigenic properties provides a highly effective immunoreagent for determining HCV infection based on its increased specificity, selectivity and sensitivity of binding to anti-HCV antibodies.

Presenting the HCV antigenic peptides as a mosaic antigen composition having the three-dimensional configuration described herein, overcomes certain disadvantages that are often encountered in using other conventional arrangements, such as in the form of recombinant fusion proteins or long-chain polypeptides. It is expected that based on the three-dimensional folding of these larger molecules, it is likely that their tertiary structure limits exposure of some epitopes on the surface of the molecule, thereby conferring lower immunoreactivity to the molecule in binding to antibodies against HCV. In contrast, the small-chain peptides of the present invention have sequences that lack extraneous amino acids which can interfere with the presentation (or exposure) of the epitopes. By immobilizing each peptide to the surface of a carrier protein, the overall surface area of each peptide is made accessible and therefore, more readily available for optimal reactivity with an incoming antibody. Accordingly, this configuration tends to increase the sensitivity of the assay.

Furthermore, immobilization of the peptides to a carrier protein through covalent linkage at their termini overcomes other known disadvantages when employing methods which involve applying the peptides directly to a solid support surface. Often this method results in lower immunoreactivity because certain antigenic peptides (e.g. peptides having less than 50 amino acid residues in length) do not bind well to solid support materials for incorporation in diagnostic assays due to their intrinsic properties. Moreover, using a particular combination and formulation of more than one different peptide makes it increasingly difficult to determine appropriate conditions that will be ideally suitable to immobilizing the group as a whole. Alternatively, the immunoreactivity of an antigenic peptide can be substantially increased through covalent coupling to a carrier protein because no peptides are excluded due to poor binding to a solid support of conventional assay devices, nor is the antigen improperly configured resulting in potential masking of the epitopes through steric hindrances. The resulting product is an antigen conjugate which comprises a large number of epitopes that are associated only with the peptides themselves. Accordingly, the improvement in assay performance is brought about not only by a combination of conjugated antigenic peptides, but also through this particular three-dimensional arrangement which enhances the overall sensitivity and specificity of binding of the peptides to anti-HCV antibodies.

In order to facility covalent linkage to the carrier protein, each HCV peptide fragment is chemically modified at either Its carboxyl or amino terminal end according to techniques well known to individuals skilled in the art. According to a preferred embodiment of the present invention, each synthetic peptide is chemically modified by the addition of a single cysteine residue at its amino or carboxyl terminal end. The peptides of the present invention are shown to be highly reactive when linked to protein carriers via cysteines located at their C-termini.

Conjugation of the peptides to the carrier protein (e.g. HSA) may be accomplished by conventional peptide linkage means using a suitable linking agent, such as: MBS (m-maleimidobenzoyl-*N*-hydroxysuccinimide ester), sulfo-MBS (*m*-maleidobenzoyl-*N*-hydroxysulfosuccinimide ester), SIAB (*N*-succinimldyl(4-Iodoacetyl)aminobrenzoate), sulfo-SIAB (sulfosuccinimidyl(4-iodoacetyl)aminobrenzoate, SMCC (succinimidyl 4-(*N*-maleidomethyl) cyclohexane-1-carboxylate), sulfo-SMCC (sulfosuccinimidyl 4-(*N*-maleido-methyl) cyclohexane-1-carboxylate), SMPB (succinimidyl 4-(*p*-maleimido-phenyl)-butyrate), sulfo-SMPB (sulfosuccinimidyl 4-(p-maleidophenyl)-butyrate), LC-SPDP (succinimidyl 6-[3(2-pyridyldithio)-propionamido] hexanoate), sulfo-LC-SPDP (sulfosuccinimidyl 6-[3(2-pyridyldithio)-propionamido] hexanoate), GMBS (N-(γ-maleimidobutyryloxy) succinimide ester), sulfo-GMBS (N-(γ-maleimidobutyryloxy) sulfosuccinimide ester), SMPT (4-succinimidyl-oxycarbonyl-alpha-(2-pyridyldithio)toluene), sulfo-LC-SMPT (sulfosuccinimidyl-6-[alpha-methyl-alpha-(2-pyridyldithio)-toluamido]hexanoate). A preferred cross-linking agent used in practicing the invention is maleimidobenzoyl-N-hydroxysulphosuccinimide ester (sulpho-MBS).

### III. Method for Detecting HCV

The mosaic antigen composition comprising a plurality of different peptides selected from the group of sequences defined in SEQ ID NO: 1, 2, 3, 4, 5 and 6 may be employed in immunoassays used to screen body fluids for the presence of anti-HCV antibodies and therefore, aid the practitioner or clinician in the diagnosis of HCV infection. Accordingly, the present invention provides an immunoassay for detecting anti-HCV antibodies in a biological test sample using the mosaic antigen composition.

Essentially, any immunoassay format which is designed to utilize the mosaic antigen composition as a capture reagent for detecting anti-HCV antibodies may be employed. In general, a biological test sample suspected of containing anti-HCV antibodies is contacted and incubated with the mosaic antigen composition for a time and under conditions which allow antibody/antigen interactions to occur. Preferably, the mosaic antigen composition is attached to a solid support. Depending upon the type of biological test sample, it may be diluted with a suitable buffer reagent, concentrated, or contacted with the solid support without any prior manipulation (or processing). For example, it usually is preferred to test serum or plasma samples which previously have been diluted, or to concentrate specimens such as urine, in order to determine the presence and/or amount of antibody present. If anti-HCV antibodies are present in the test sample, they will form an immune complex (i.e. antigen-antibody complex) with the mosaic antigen composition and become attached (or bound) to the solid support. The solid support is subsequently washed with a buffer solution to remove any unbound and irrelevant components. After washing, the immune complex is detected with an indicator reagent and allowed to incubate for a time and under conditions for a second complex to form. Immune complexes are detected by any of a number of known techniques, depending on the format of the assay. For example, a conjugate of an anti-mammalian immunoglobulin labeled with a signal-generating component is commonly employed as an indicator reagent. Various signal-generating components may be employed to assist in the detection of immune complexes which are formed, as is conventional in the art. Thus, suitable signal-generating components include, without limitation, enzymes, radionuclides, luminescent, fluorescent and chemiluminescent moities, magnetic particles and directly visible particles, such as colloidal gold. The presence of an immune complex confirms the presence of antibodies to HCV in the test sample and is determined by measuring the signal generated. As with many indicator reagents, the amount of antibody present is usually proportional to the signal generated. The presence of the immune complex in the test sample may be detected visually or instrumentally using, for example, an automated scanning and interpretation device.

Accordingly, a further aspect of the present invention provides a method for *in vitro* diagnosis or detection of anti-HCV antibodies present in a subject comprising the following steps:
- contacting a biological test sample with a mosaic antigen composition comprising a plurality of different peptides defined by SEQ ID NO: 1, 2, 3, 4, 5 and 6; and
- detecting an immunological complex formed between antibodies to HCV in said biological sample and said peptide composition, characterized in that the presence of said complex being indicative of the presence of antibodies to HCV in said biological sample.

### IV. Diagnostic Test Kit

The present invention also provides a test kit for carrying out an immunoassay which contains the mosaic antigen composition as an essential component. Accordingly, the kit will normally contain, in suitable containers, the mosaic antigen composition, control antibody formulations (positive and/or negative), specimen diluent and/or washing buffer, and an indicator reagent. If the indicator reagent has an enzyme as the signal-generating component, then the kit also contains a substrate for the enzyme if it does not generate a signal directly. The mosaic antigen composition is preferentially immobilized to a solid support which is immunologically compatible with the mosaic antigen composition. Instructions for carrying out the assay generally will be included in the kit.

Therefore, another aspect of the present invention provides a diagnostic test kit for determining the presence of anti-HCV antibodies in a biological test sample, comprising:
- a mosaic antigen composition comprising a plurality of different peptides defined by SEQ ID NO: 1, 2, 3, 4, 5 and 6, wherein the mosaic antigen composition is preferably immobilized to a solid support;
- an indicator reagent, preferably consisting of a mammalian anti-human immunoglobulin or staphylococcal protein A labeled with colloidal gold;
- control standards;
- a specimen diluent and/or washing buffer for removing unbound material; and
- instructions for carrying out the assay.

In a preferred embodiment of the invention, the type of immunoassay employed is based on a capture antibody assay format using an immunofiltration dotting assay system for the purposes of convenience and utility wherein one or more species of the mosaic antigen composition has been immobilized onto a solid support to effect a reaction. Immunofiltration dotting techniques are well known in the art. For example, a specimen sample is added to a panel of the mosaic antigen composition placed in an array on a solid support. Preferably, the solid support is a nitrocellulose membrane which is secured and protected by a test cartridge and attachment of the composition to the support is through passive adsorption or covalent coupling. The prepared solid support is contacted with a test sample effecting capture of any anti-HCV antibodies which may be present in the sample. If any antibodies are present, they will recognize and bind to the mosaic antigen composition resulting in the formation of an immune complex (antigen-antibody). The captured antibodies are detected by reaction of the immune complex with an indicator reagent which is preferably an anti-human immunoglobulin or protein A labeled with a component generating a colorimetric signal, such as colloidal gold or dyed polystyrene microparticles or other types of visally detectable dyes.

To aid in understanding the invention, several terms are defined below.

**Antibody** refers to a family of glycosylated proteins called immunogloblins, which can specifically combine with an antigen. Hence, the term "antibody" is a functional term, and may also be the plural form "antibodies."

**Antigen** refers to a compound which is recognized by a specific antibody.

**Biological test sample** refers to a biological fluid which is the possible source of the antibodies of interest. These components are well known in the art and include biological test samples which can be tested by the methods described herein. Examples include, without limitation, human and animal body fluids such as whole blood, serum, plasma, cerebrospinal fluid, urine and lymph fluids.

**Capture reagent or immunoreagent** refers to an antigen which can interact with the analyte of interest (i.e. antibody) and which allows physical discrimination (or separation) between the analyte and irrelevant compound or molecule found in the sample to analyse. Usually the capture reagent is directly or indirectly attached (or immobilized) to a solid support material, thereby enabling the separation of the analyte from the test sample.

**Carrier component** includes any naturally occurring or synthetic polypeptide, protein or polymer having a molecular weight greater than about 5,000 daltons. Representative carrier proteins include bovine serum albumin (BSA), keyhold limpet hemocyanin (KLH), human serum albumin (HSA), poly-L-lysine, bovine gamma globulin, cytochrome C and other similar macromolecules.

**Epitope** refers to that portion of an antigen which is specifically recognized by an antibody. It is also referred to as the antigenic determinant site.

**Immune complex** refers to the combination formed when an antibody binds to an epitope on an antigen.

**Indicator reagent** comprises a signal-generating component capable of generating a detectable signal by external means which, in turn, is conjugated to a molecule that can specifically bind to one of the member of the immune complex to be detected.

**Recombinant fusion protein** is a polypeptide in which viral antigen(s) form part of a single continuous chain of amino acids and therefore, does not occur in nature. The viral antigens may be connected directly to each other by peptide bonds or be separated by intervening heterologous amino acid sequences.

### BRIEF DESCRIPTION OF THE DRAWINGS

In the following description, the invention will be explained in detail with the aid of the accompanying figures which illustrate preferred embodiments of the present invention and in which:
Figure 1 illustrates the distinct regions of the polyprotein encoded by the HCV genome (Japanese isolate) comprising the core protein (C), the major envelope protein (E1), the envelope protein/non-structural protein (E2/NS1), and non-structural proteins (NS2, NS3, N4A, N4B and N5) (Kato, N. *et al.* (1990) Proc. Natl. Acad. Sci. USA, 87:9524-9528); and
Figure 2 shows the amino acid sequence within the core region of the HCV genome and the location of each of the six peptides, identified as MDL-1 to MDL-6, within the sequence.

The further scope and applicability of the present invention will become apparent from the detailed description and examples given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description and these examples.

### DETAILED DESCRIPTION

### I. Antigenic Peptides and Combinations

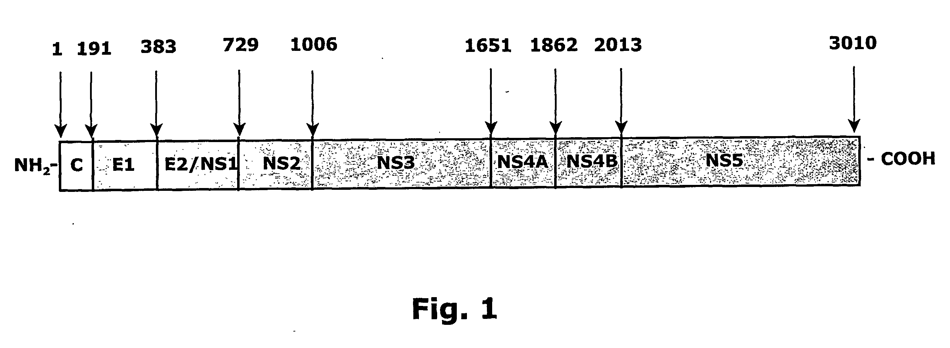

In order to develop a specific and sensitive immunoassay which will enable a reliable diagnosis of hepatitis C virus (HCV) infection to be made, it is important to first identify the HCV immunodominant regions that are beneficial in screening for this disease. The putative HCV polyprotein encoded by the HCV genome comprises 7 distinct regions which can potentially contain useful diagnostic markers for HCV infection. These regions are the core (C) and envelope (E1 and E2/N1), which encode for structural proteins, and the NS2, NS3, NS4A/NS4B and the NS5 regions (Kato, N. *et al*. (1990) Proc. Natl. Acad. Sci. USA 87, 9524-9528), which encode for non-structural proteins. Figure 1 illustrates the organization of the HCV genome which encodes a single polyprotein that is cleaved to generate both structural and non-structural proteins.

Extensive investigative research of both the structural and non-structural regions of the HCV genome has determined that there are approximately 200 different antigenic determinants suitable for utilization in HCV diagnostics based on screening methods conducted to evaluate their immunochemical reactivity.

In particular, previous studies have shown that peptides derived from the core region are highly suitable for HCV diagnostics based on results obtained with panels of human samples (Jackson *et al*. (1997) J. Med. Virol. 51, 67-79; Hosein *et al*. (1991) Proc. Natl. Acad. Sci. USA 88, 3647-3651; Nasoff *et al*. (1991) Proc. Natl. Acad. Sci. USA 88, 5462-5466; Shirai *et al*. (1994) J. Virol. 68, 3334-3342; see Cuthbert, J. (1994) Clin. Microb. Reviews 7, 505-532 for a comprehensive review). In addition, it has been discovered using seroconversion panels, that using peptides from the core region also shortens the "window period" for detection of anti-HCV antibodies in sera of infected individuals (Hosein *et al*. (1991) Proc. Natl. Acad. Sci. USA 88, 3647-3651; Cuthbert, J. (1994) Clin. Microb. Reviews 7, 505-532). Besides containing several imunodominant regions, or epitopes, the HCV core/capsid region sequence is highly conserved and thus possesses a high degree of sequence homology between the different virus strains. On this basis, it has been postulated that antigens or peptides derived from the core region are good candidates for anti-HCV diagnostics and useful for the development of a clinical test which gives minimal or no discrimination between the different HCV genotypes.

The present invention provides six different antigenic peptides that are derived essentially from the core region which is believed to define the nucleocapsid protein of HCV. The core region extends from the N-terminal of the HCV polyprotein to approximately amino acid 191 as indicated in Figure 1. The amino acid sequence shown in Figure 2 is the sequence of the HCV JK1G isolate.

These novel peptides are useful for the diagnosis of HCV infection and contain amino acid sequences which possess several successive epitopes within the nucleocapsid protein that are highly reactive with antibodies specific to HCV. The antigenic properties of each short peptide was comparatively analyzed among a number of possible candidates to determine the best peptide sequences for detection of human anti-HCV antibodies found in sera of infected patients. Accordingly, it has been determined by the inventor that the combination and selection of the six peptides described herein which span a region of the core protein between positions 1 to 80 are highly immunoreactive with anti-HCV antibodies found in sera of infected individuals. A panel of overlapping peptides between positions 1 and 186 was synthesized and tested for Immunoreactivity with a well characterized human serum panel. However, based on the results, it was determined that all of the immunoreactivity was concentrated within the region between positions 1 to 80.

The antigenic peptides have the amino acid sequences defined as follows:

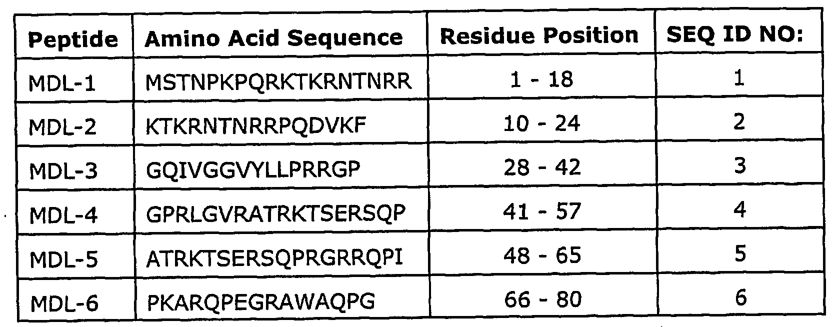

Figure 2 illustrates how these sequences span the amino acid residue positions of the HCV polyprotein encoded by the core region of the HCV genome.

Each of the six peptide sequences overlaps with its sequential or neighboring sequence to an extent that varies anywhere in length from about 2 to 10 amino acid residues. Therefore, the peptides collectively contain sequences where epitopes have been already been found to exist. It has been discovered by the inventor that the overlapping of regions between all of the six peptides demonstrates a higher immunochemical reactivity when used in combination with one another as opposed to using a single sequence. Furthermore, it has been discovered that the six different peptides of the present invention show high sensitivity in detecting antibodies directed against the core region of the HCV polyprotein. An advantage of this reactivity is that the particular selection and combination of the six antigenic peptides according to the invention greatly increases the specificity and the sensitivity of the immunological assay, thereby enabling a more reliable diagnosis of HCV infection to be determined. Antibodies that exist in response to a range of HCV variants are more likely to be detected using a combination of antigenic peptides in contrast to a protein or polypeptide having only a single epitope.

To determine the efficacy of the subject peptides in detecting HCV antibodies, the peptides were tested for their immunoreactivity with specimens previously selected through the screening of thousands of patient with HCV and normal sera. Information regarding the HCV-specific serum panels are provided in the Examples.

The peptides range in length from 15 to 19 amino acid residues which have also been determined to work optimally as capture antigen in the design and development of the mosaic antigen composition. Following immobilization to a carrier component, there is an increased tendency for peptides having a length greater than 20 amino acid residues to acquire aberrant three dimensional structures due to improper folding or interaction with the carrier component which is likely to result in reduced exposure of linear antigenic determinants. On the other hand, it is expected that selecting peptides less than 12 amino acid residues in length will likely compromise their immunoreactivity due to the limited ability of an antibody to effectively recognize and bind to an antigenic determinant having less than a required minimal number of amino acid residues.

The novel peptides can be prepared by methods known in the art including chemical synthetic methods and recombinant DNA technology. Representative peptides used in the practice of the invention which contain antigenic domains of the HCV nucleocapsid protein are preferably synthetically derived peptides having the amino acid sequences defined by SEQ ID NO: 1, 2, 3, 4, 5 and 6. Accordingly, the peptides have been prepared through chemical synthesis using automated solid-phase methods so that it is possible to avoid problems associated with using recombinant technology. Using this method provides a reproducible antigenic peptide of high integrity with consistent yields and as a result, reproducibility of the test results can be assured over those methods which derive antigens by recombinant technology.

The peptides of the present invention may be prepared using standard chemical synthesis techniques that are known to those skilled in the peptide art, such as the Merrifield solid phase synthesis technology (Merrifield, J. Am. Chem. Soc. 85:2149-2154, 1963). Other examples of the many techniques available may be found in J. M. Stewart and J. D. Young, "Solid Phase Peptide Synthesis", W.H. Freeman Co., San Francisco, 1969; J. Meienhofer, "Hormonal Proteins and Peptides", vol. 2, p 46., Academic Press (New York), 1973 for solid phase peptide synthesis; E. Schroder and K. Lubke, "The Peptides", Vol. 1, Academic Press (New York), 1965 for classical solution synthesis, "Synthetic Peptides: A User's Guide", W.H. Freeman & Co. (New York), 1992; and Jones, "The Chemical Synthesis of Peptides", Clarendon Press, Oxford, 1994.

In general, these methods comprise the sequential addition of one or more amino acids or suitably protected amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final polypeptide.

According to another aspect of the present invention, there is provided a particular composition of different peptides selected from the group of amino acid sequences defined by SEQ ID NO: 1, 2, 3, 4, 5 and 6. The use of a peptide composition of the subject peptides provides the mosaic antigen composition with a broader spectrum of immunoreactivity against a greater number of HCV strains, thereby enhancing the ability of the antigenic peptides to react with antibodies specific for a wider range of HCV variants. The various combinations of the subject peptides have been tested for their immunoreactivity in detecting anti-HCV antibodies through extensive experimentation, the results of which are provided in the Examples. The peptide compositions of the present invention may be comprised of one or more of peptides MDL-1, MDL-2, MDL-3, MDL-4, MDL-5 and MDL-6 (i.e. SEQ ID NO: 1, 2, 3, 4, 5 and 6, respectively). In a preferred embodiment of the present invention, the peptide compositions include a combination of all six peptides MDL-1, MDL-2, MDL-3, MDL-4, MDL-5 and MDL-6 for detecting and diagnosing HCV. Moreover, effective ratios of the subject peptides in compositions typically range from about 0.5 to about 8.0 on a peptide to carrier mass ratio. More preferably, the combination of peptides range from about 1.0 to 4.0 on a peptide to carrier mass ratio. An especially preferred peptide composition for the detection and diagnosis of HCV infection is a mixture of peptides MDL-1, MDL-2, MDL-3, MDL-4, MDL-5 and MDL-6 in a peptide to carrier mass ratio of about 4:1:4:4:1:2, respectively.

### II. Mosaic Antigen Composition

According to an important aspect of the present invention, optimal immunoreactive performance is obtained when the subject peptides are immobilized individually through orientation specific covalent linkage by their terminal end to a carrier component, as opposed to their use as free peptides. In this way, full exposure of each antigenic domain is exploited for recognition and binding to anti-HCV antibodies. It has been determined through experimentation that the carrier-linked composition of peptides results in higher positive/negative test ratios in the detection of anti-HCV antibodies than free peptide compositions. On this basis, the carrier-linked peptides demonstrate superiority over free peptides when employed as an immunoreagent in rapid immunodiagnostic assays.

Suitable carrier components include proteins, polypeptides or polymers, providing they do not exhibit cross-reactivity with other antibodies in human serum and therefore, adversely affect the overall sensitivity and specificity of the assay. Preferably, macromolecules used for conjugation to the synthetic peptides of the invention include any naturally occurring or synthetic polypeptide or protein molecules having a molecular weight greater than about 5,000 daltons which are not immunoreactive with a test sample to be analyzed and which do not significantly contribute to or detract from the reactivity of the synthetic peptides.

Thus, a carrier protein is a macromolecule that is non-reactive in the diagnostic analysis for antibodies to HCV and that bears functional groups permitting the synthetic peptides to be chemically linked to its surface. The resulting conjugate can then be immobilized, either directly or indirectly, to a solid substrate for subsequent use in a diagnostic assay. Carrier proteins commonly used include albumins, globulins and hemocyanins, all of which can be obtained from many sources. Representative carrier proteins include bovine serum albumin (BSA), keyhold limpet hemocyanin (KLH), human serum albumin (HSA), poly-L-lysine, bovine gamma globulin, and other similar polypeptide molecules. Also included is the avidin/biotin bridging system wherein the peptides can be complexed with biotin via their C- or N-termini and can be immobilized on avidin or streptavidin adsorbed on the solid surface ["Chemistry of Protein Conjugation and Cross-linking", S. C. Wong, CRC Press (1991)].

According to a preferred embodiment of the invention, optimum assay performance overall is achieved when HSA is selected as the carrier protein compared to other proteins. Certain advantages afforded by its use are that it is a carrier with high density labeling due to possession of a high number of lysine residues at the surface of the protein. Overall, this makes HSA simpler to chemically modify with a heterobifunctional cross-linker and it remains soluble even following peptide conjugation. Moreover, since HSA is of human origin, carrier-induced epitopic reactions due to foreign or diet-induced antibodies in circulation are avoided. These types of reactions are known to be elicited by proteins derived from foreign sources and thus, are potentially able to generate false positive reactivities when diagnosing HCV infection. In this regard, developing a mosaic antigen composition which employs HSA as the carrier protein facilitates their use in membrane-based rapid assays using flow-through or lateral flow assay platforms.

An alternative means of immobilizing the peptides through orientation specific covalent linkage is to couple them directly to a preactived solid support surfaces. For example, immobilization of the peptides may be accomplished using a solid support surface having certain chemical properties or specificities, such as having exposed reactive groups which will facilitate peptide linkage. Depending on the type of preactivated solid support surface used (e.g. microwells, microspheres, membrane), the subject peptides may or may not have to be functionalized with an appropriate reactive group at their terminal end in order to carry out a reaction with these surfaces. Among such surfaces having exposed reactive groups are those preferably having a --COOH, --CHO, NH₂, --OH group, or a vinyl group containing a double bond --CH=CH₂ which can be activated to give especially --CHO, --COOH, --NH₂, or --OH groups. Such surfaces are commercially available (e.g. Covalink™ NH surface, ProActive® microspheres, Cortex Biochem Magabeads™, Estapor® (Bang Laboratories, Inc.) , Dynal®) in various forms having at their surface --COOH, --NH₂, --OH, or other reactive groups.

Advantageously, the subject peptides are chemically coupled to a carrier component due to the flexibility afforded by this particular format. For example, a higher density of the mosaic antigen composition thus produced is easier to characterize and manipulate in that it can be concentrated in a localized region of a test zone of an immunoassay device. In this regard, the overall cost of production can be significantly reduced during upscale manufacturing since a lesser amount of the immunoreagent may be employed in a rapid type immunodiagnostic device. Alternatively, using a chemically-active solid support surface entails using a larger region of a test zone in order to achieve suitable immunoreactive performance and thus, is less preferred in the practice of the invention.

There are many well described techniques for coupling peptides to carrier proteins. Depending on the types of peptides used, the linkage may occur at the N-terminus, C-terminus or at an internal site in the peptide. The peptide may also be derivatized for coupling. Detailed descriptions of a wide variety of coupling procedures are given, for example, in Van Regenmortel, M.H.V., Brian, J.P., Muller, S., and Plaue, S., Laboratory Techniques in Biochemistry and Molecular Biology, Vol. 19, Synthetic Polypeptides as Antigens, Elsevier Press, Amsterdarn, N.Y., Oxford, 1988.

According to a preferred embodiment of the present invention, each HCV peptide is chemically modified at its carboxyl terminal end to facility covalent linkage to a carrier protein. In order to facilitate conjugation to the carrier protein, an amino acid residue is added to the carboxyl terminal end of each peptide which is not already provided in any of the sequences of the original peptides defined by SEQ ID NO: 1, 2, 3, 4, 5 and 6. The same amino acid residue is added to the carboxyl terminal end of each peptide in order to provide the appropriate functional group which will participate in the covalent bond between the peptide and the protein carrier. Amino acids such as cysteine, methionine, arginine, tyrosine, lysine, glutamic acid, aspartic acid and derivatives thereof are useful for grafting onto the carboxyl terminus of the synthetic peptides in providing the appropriate functional group for coupling to a protein carrier. In the invention, a cysteine residue is selected as the carboxyl terminal amino acid which participates in the conjugation of each peptide to the surface of a protein via a single bond.

A cysteine residue added at the carboxyl terminal end of a peptide has been found to be particularly useful for forming conjugates with --SH reactive groups such as maleimide functional group. The --SH groups on the synthetic antigen (incorporated as cysteinyl or homocysteinyl residues on or in the polypeptide chain) will be reacted with suitable carriers which have been activated with functional maleimide groups. The --SH function may be incorporated into the antigen by either incorporation of a Cys (or homo-Cys) residue in -amino acid linkage, or by reaction of an amino function (-amino or ,-amino of lysine) with cysteine thiolactone. Alternatively, the --SH function on the antigen may be activated as the 2- or 4-thiopyridyldisulfide and bonded to the --SH groups on a suitable carrier. This sequence may also be reversed with the carrier --SH activated as the thlopyridyldisulfide.

Numerous reagents and procedures for conjugating peptides to carrier protein molecules are well known in the art and can be used in making the mosaic antigen composition of the present invention. The components to be conjugated, the peptides and protein carrier, typically contain one or more of one or more of the following functional groups which can be used for conjugation: thiol groups, amino groups, carboxyl groups, and hydroxyl groups, phosphate groups or sulfo groups. However, if appropriate functional groups are lacking, they may be introduced by methods well known to the art. Such methods include the use of homobifunctional and heterobifunctional cross-linking molecules.

Two molecules functionalized with thiol groups may be conjugated by oxidizing the thiols to disulfides. Alternatively, such molecules may be conjugated by linking the thiols with a homobifunctional reagent, such as a bis-maleimide or a bishaloacetyl compound. Two molecules functionalized with amine groups can be conjugated by use of homobifunctional reagents such as gluteraldehyde or disuccinimidyl esters. However, better control of a conjugation process may often be attained by utilizing a heterobifunctional reagent. For example, a molecule functionalized with thiol groups can be conjugated to a molecule functionalized with amine groups by means of a heterobifunctional reagent that possesses both maleimide and succinimidyl ester functions. Examples of heterobifunctional linkers include succinimidyl 4-(N-maleimidomethyl) cyclohexane-1-carboxylate (SMCC), succinimidyl 4-(iodoacetyl)aminobenzoate (SIAB) and succinimidyl 4-p maleimidophenyl) butyrate.

It has been determined that the synthetic peptides of the invention retain a high degree of immunoreactivity when chemically bound via their carboxy-termini (C-termini) to carrier proteins. Conjugation of the peptides to a carrier protein may be accomplished by conventional peptide linkage means using a suitable linking agent, such as p-maleimidobenzoic acid, p-methyldithlobenzoic acid, maleic acid anhydride, succinic acid anhydride, gluteraldehyde, or other similar linking agents.

More preferably, conjugation of the carrier protein (e.g. HSA) with the subject peptides in which a carboxyl terminal cysteine has been introduced, may be accomplished by conventional peptide linkage means using a suitable linking agent, such as: MBS (m-maleimidobenzoyi-N-hydroxysuccinimide ester), sulfo-MBS (m-maleidobenzoyl-N-hydroxysulfosuccinimide ester), SIAB (N-succinimidyl(4-iodoacetyl)aminobrenzoate), sulfo-SIAB (sulfosuccinimidyl(4-iodoacetyl)aminobrenzoate, SMCC (succinimidyl 4-(N-maleidomethyl) cyclohexane-1-carboxylate), sulfo-SMCC (sulfosuccinimidyl 4-(N-maleido-methyl) cyclohexane-1-carboxylate), SMPB (succinimidyl 4-(p-maleimido-phenyl)-butyrate), sulfo-SMPB (sulfosuccinimidyl 4-(p-maleidophenyl)-butyrate), LC-SPDP (succinimidyl 6-[3(2-pyridyldithio)-propionamido] hexanoate), sulfo-LC-SPDP (sulfosuccinimidyl 6-[3(2-pyridyldithio)-propionamido] hexanoate), GMBS (N-(γ-maleimidobutyryloxy) succinimide ester), sulfo-GMBS (N-(γ-maleimidobutyryloxy) sulfosuccinimide ester), SMPT (4-succinimidyl-oxycarbonyl-alpha-(2-pyridyldithio)toluene), sulfo-LC-SMPT (sulfosuccinimidyl-6-[alpha-methyl-alpha-(2-pyridyldithio)-toluamido]hexanoate). A preferred cross-linking agent used in practicing the invention is maleimidobenzoyl-N-hydroxysulphosuccinimide ester (sulpho-MBS).

### III. Assay Formats Using the Mosaic Antigenic Composition

The mosaic antigen composition provided herein may be utilized in essentially any immunoassay format that employs a HCV-associated antigen as capture reagent, as a means to detect antibodies against HCV. Based on the physical format of the assay and the type of indicator or imaging reagent used, there are several known variations in the overall design of an immunoassay which are established on the basic principle of specific binding members (e.g. antigen and antibody).

Many assay configurations are practiced and well known to the individual skilled in the art. Using the mosaic antigen composition according to the present invention, these assays can be adapted to detect anti-HCV antibody and are contemplated to be within the scope of this invention (Portsman, T. and S.Y. Kiessig, 1992, "Enzyme Immunoassay Techniques: An Overview", Journal of Immunological Methods 150:5-21; Hamilton, R.G. and N.F. Adkinson, 1988, "Quantitative Aspects of Solid Phase Immunoassays" In: ELISA and Other Solid Phase Immunoassays, (D.M. Kemeny and S.J. Challacombe, Eds.), pp. 57-82, John Wiley and Sons Ltd., Chichester; Tijssen, P., 1985, "Outline of the Strategies for Enzyme Immunoassays" In: Practice and Theory of Enzyme Immunoassays (R.H. Burdon and P.H. van Knippenberg, Eds.) pp. 9-20 Elsevier, Amsterdam).

In general, a method of using the mosaic antigen composition for detecting anti-HCV antibody involves contacting the mosaic antigen composition with a biological test sample suspected of containing anti-HCV antibodies to form a mixture. The mixture is incubated under conditions and for a time which will permit the HCV specific antibodies present in the sample to bind to mosaic antigen composition thereby forming an immune complex (antigen-antibody complex). The formation of an immune complex between the antibodies from the sample and the mosaic antigen composition is an indication that HCV antibodies are present in the sample. Immune complexes thus formed are detected by any number of known techniques, depending on the type of assay format employed. Suitable techniques generally involve the addition of an indicator reagent comprising a signal-generating component that is capable of generating a measurable signal and which is attached to a secondary binding member (e.g. anti-human antibody or staphyloccocal protein A) for specific recognition of the anti-HCV antibodies bound the mosaic antigen composition. The immune complex and indicator reagent are incubated for a specific time and under specific conditions to allow detection with adequate sensitivity and specificity. Types of signal-generating components that are commonly used in indicator reagents include enzymes, fluorescent probes, chemiluminescent substrates and probes, radioactive markers, metal colloids, colored microspheres and fluorescent microspheres. For example, assays which generate a signal from the immunological complex using enzyme-labeled antibodies or proteins are referred to as ELISA (enzyme-linked immunosorbent assays) which are suitable for rapid and large scale clinical screening of serum of patients, blood for transfusion or blood derivatives. Moreover, several well known strategies can be used to amplify the signal in ELISA in order to improve the sensitivity and limit of detection. These strategies include the use of avidin-biotin bridging system, substrate cascade reactions and enzyme/anti-enzyme antibody conjugates, and the like.

The type of immunoassay performed in which the mosaic antigen composition is utilized may be, without limitation, either competitive or non-competitive version of homogenous or heterogenous formats. In a heterogeneous format, the mosaic antigen composition is typically bound to a solid support to facilitate capture of the analyte from the test sample and to facilitate removal of non-specifically bound substances which may cause interference. The types of solid supports that can be used are described below. A classical example of heterogeneous format is the capture antibody assay format in which the mosaic antigen composition is attached to a solid support by passive adsorption or by covalent chemical coupling. This assay format is commonly used for antibody detection. The solid support is contacted with the test sample and if anti-HCV antibody is present, it will react with the mosaic antigen composition to form an immune (antigen-antibody) complex. The solid support is washed with a solution to remove any nonspecifically bound material to ensure adequate specificity of the test. The immune complex, which remains bound to the solid support, can be detected by the addition of an indicator reagent. the indicator reagent can be an anti-human antibody or staphyloccocal protein A linked to a signal-generating component which can generate a signal when the indicator reagent binds to the immune complex. Depending on the type of indicator reagent used, specific types of sandwich assays widely used by those skilled in the art include enzyme-linked immunosorbent assays (ELISA), fluorescent immunoassays (FIA) and radioimmunoassays (RIA assays), to name a few.

Alternatively, the assay may be in a homogeneous format, in which a solution resulting from incubation of the biological test sample with the mosaic antigen composition does not require separation of both the captured and free antibody before detection of HCV antibodies in a sample. Agglutination is the most common example of a homogeneous format. In this assay format, the mosaic antigen composition would have to be immobilized onto high refractive index micro-particles, such as a latex bead, which is then incubated with the test sample. If any anti-HCV antibody is present, it will bind with the mosaic antigen composition causing agglutination (or clumping) of the microparticles. Agglutination can be detected either visually or spectrophotometrically for turbimetric analysis.

Alternative assay formats for detection of anti-HCV antibodies are the competitive versions of both heterogeneous and homogeneous assay formats. An example of competitive heterogeneous assay format for detection of anti-HCV antibodies using the mosaic antigen composition is the competitive antibody capture assay format. In this format, the anti-HCV antibody (analyte) is measured by its ability to compete with a labelled anti-HCV antibody, such as a anti-HCV core protein polyclonal antibody labelled with an enzyme. The extent of competition can be proportional to the analyte concentration within a specified analytical range. An example of a competitive version of a homogeneous assay would be the competitive agglutination assay format. In this format, the anti-HCV antibody (analyte) is measured by its ability to compete the binding of the mosaic antigen composition in solution with a anti-HCV core protein polydonal or monoclonal immobilized on polystyrene microparticles antibody. The competition would result in decreased agglutination. The extent of competition in agglutination can be correlated to the analyte concentration as in the case of the heterogeneous assay format.

Solid supports advantageous for immobilizing the mosaic antigen composition of the present invention are well known to those skilled in the art. A suitable support will depend upon the type of immunoassay format that is performed however, it is desired that the type of support chosen will have suitable characteristics for macromolecule immobilization and not interfere with the immunoreactivity of the antigen composition nor the production of a detectable signal from the signal-generating component. Examples of solid supports include microtiter plates, test tubes, microparticles (e.g. polystyrene, glass or magnetic), nitrocellulose and nylon membranes, plastic, derivatized plastic, metal and silicon, and others.

The signal-generating component used as a means to detect the formation of an immunological complex through antigen/anti-HCV antibody binding will allow visual detection of a precipitate or dye/particle accumulation (e.g. colloidal gold/latex microparticles), or instrumentation-based detection such as spectrometry, fluorometry, radiometry, or the like. Examples of signal-generating components include, without limitation, fluorescent compounds (e.g. fluorescein, rhodamine and phycoerythrin), luminescent compounds, radioactive elements, enzymes (e.g. alkaline phosphatase, horseradish peroxidase and beta-galactosidase) used with chromogenic, fluorogenic, or luminescent substrates, magnetic particles, and directly visible particles (e.g. colloidal gold). These signal-generating components can be used in combination with the avidin/biotin bridging system for improving the limit of detection and versatility of the assay.

In a preferred embodiment of the present invention, the biological test sample suspected of containing anti-HCV antibody is contacted with a solid support to which the mosaic antigen composition has been immobilized. Immobilization is achieved by passive adsorption or covalent linkage with a chemically activated support to allow optimal exposure of mosaic antigen composition on the surface of the solid support in order to promote specific antibody recognition. A preferred solid support material for immobilization of the mosaic antigen composition is membrane-based support suitable for use in lateral and flow-through assay devices. This includes any porous membrane or material through which a test sample can easily migrate or diffuse by capillary action. Examples of suitable materials include nitrocellulose, nylon, polypropylene fiberglass and cellulose acetate. The selection of individual (or combination) of membrane components is based according to physical and biochemical characteristics of the test sample being assayed. Fluidity, antibody concentration and titer, protein concentration, presence of potential interfering substance such as lipids and cross-reactive immunoglobulins are important factors that can dictate the use of specific support in order to achieve optimal performances. The sample to be tested is incubated for a specified time period and under specific conditions with the antigen-coated solid support to allow immune recognition. If any anti-HCV antibody happens to be present in the test sample, binding will occur with the mosaic antigen composition to form an immune complex and therefore, become affixed to the solid support. The solid support is washed with a suitable buffer reagent to remove any non-specifically (or irrelevantly) bound material. Any specific immune complexes, which are formed are then are, detected with an indicator reagent comprising anti-human antibody or protein A conjugated to a signal-generating component. This mixture is incubated for a specified time period and under specific conditions sufficient to allow recognition between the labeled anti-human antibody and the anti-HCV antibody (sample), bound to the immobilized mosaic antigen composition. The presence of any anti-HCV antibody in the test sample is thus determined by measuring a signal generated from the indicator reagent, either visually or instrumentally, wherein within a specified antibody concentration range, the concentration of antibody in the sample is proportional to the amount of signal generated. Standards and controls are treated in the same manner as the test sample. Depending upon the type of biological test sample used in determining HCV infection, it may be diluted with a suitable buffer reagent, concentrated, or contacted with the solid phase without any manipulation. For example, prior to testing the biological sample, it usually is preferred to initially dilute serum or plasma samples, or concentrate specimens such as urine, in order to allow the sample to be tested within the working range of the assay and thus, accurately determine the presence and/or amount of antibody present.

### IV. Diacinostic Test Kit

The mosaic antigen composition described herein will typically be packaged in the form of a diagnostic kit for use in the detection of HCV infection. The kit will normally contain, in separate containers, the mosaic antigen composition, positive and negative control samples, an indicator reagent, such as a labeled anti-human antibody and an ancillary signal generating component (e.g. enzyme substrate) if the indicator reagent does not generate a signal directly. As described herein, the term "separate container" refers to any material such as glass, plastic (e.g., polyethylene, polypropylene or polycarbonate), paper, foil and the like capable of holding within fixed limits the mosaic antigen composition of the present invention, as well as other components provided in the diagnostic test kit. Thus, for example, a separate container can be a glass vial used to contain a predetermined amount of the mosaic antigen composition or it can also be a well of a microtiter plate to which the mosaic antigen composition has been immobilized, i.e. covalently linked so that it is capable of being immunologically bound by an antibody. Instructions for carrying out the assay generally will be included in the kit. Such instructions generally describe the immunoreagent concentration or at least one assay method parameter such as the relative amounts of immunoreagent and sample to be admixed, time periods for immunoreagent/sample admixtures, temperature, buffer conditions and the like.

The indicator reagent should comprise a signal-generating component capable of indicating the formation of an immune complex between anti-HCV antibodies and the mosaic antigen of the present invention. The indicator reagent of the diagnostic test kit can be provided in a liquid or a lyophilized form. Where the indicator reagent utilizes an enzyme as the signal-generating component, the enzyme's substrate can also be provided in a separate container of the diagnostic test kit.

The signal-generating component used as a means to detect the formation of an immune complex through antigen-anti-HCV antibody binding will allow visual detection of a precipitate or a color change, or automated detection by fluorometry, spectrophotometry, radiometric measurement, or the like. Examples of signal-generating components include, without limitation, fluorescent compounds (e.g. fluorescein, rhodamine and phycoerythrin), luminescent compounds, radioactive elements, enzymes (e.g. alkaline phosphatase, horseradish peroxidase and beta-galactosidase) used with chromogenic, fluorogenic, or luminescent substrates, magnetic particles, and directly visible particles (e.g. colloidal gold). These signal-generating components can be used in combination with the avidin/biotin bridging system for improving the limit of detection and versatility of the assay.

If desired, the mosaic antigen composition of the present invention can be typically immobilized to a solid support material by passive adsorption or through covalent linkage using a chemically activated support (surface). Several antigen immobilization protocols and strategies are available and well known to those skilled in the art. Solid supports advantageous for immobilizing the mosaic antigen composition of the present invention are well known to those skilled in the art. A suitable support will depend upon the type of immunoassay format that is performed and the physical and chemical characteristics of the mosaic antigen composition. However, it is desired that the type of support chosen will not interfere with the immunoreactivity of the antigen composition nor the production of a detectable signal from the signal-generating component. Examples of solid supports include polystyrene microtiter plates, test tubes and microparticles (e.g. polystyrene, glass or magnetic microspheres, nitrocellulose and nylon membranes, etc.).

In a preferred embodiment of the invention, the lmmunofiltration dotting assay system is employed for use of the mosaic antigen composition in a diagnostic kit. Briefly, the immunofiltration dotting assay system utilizes a panel of the mosaic antigen composition placed in an array on a nitrocellulose solid support which is secured within a test cartridge. The mosaic antigen composition is confined within a specific reaction zone through immobilization to the surface of the nitrocellulose membrane surface. Immobilization is achieved by passive adsorption of the carrier protein-peptide conjugates allowing the antigenic peptides to be immobilized in a favorable orientation for antibody recognition to prevent leakage during the test procedure. The test sample is applied on the HCV antigen-dotted nitrocellulose membrane allowing any anti-HCV antibodies present in the sample to be captured by the immobilized antigens. After the reaction takes place, the test cartridge is washed with a buffer to remove unbound materials and any immune complexes formed through antigen-antibody reactions are identified using suitable well-known detector reagents. A preferred detector reagent is an anti-human immunoglobulin antibody to which a signal-generating component is coupled, preferably one which is visually detectable such as colloidal gold or dyed polystyrene microparticles. Preferably, the detection could be quantified using an instrument for colorimetric densitometry using reflectance or image analysis technologies.

### V. Vaccine Composition

In addition to their usefulness as immunodiagnostic reagents, the HCV mosaic antigen composition described herein may be potentially utilized as an immunogen to stimulate production of antibodies in healthy mammals, including humans. The method comprises introducing an effective amount of the peptide composition, including a mixture of these six peptides conjugated to a carrier component which is compatible for vaccination purposes (i.e. to raise a specific antibody response to HCV), into the body of a healthy mammal by intraperitoneal or subcutaneous injection.

In addition, animals may be immunized to raise polyclonal antibodies to the diagnostic peptide epitopes or to produce an immune response in mice in preparation for hybridoma technology to prepare monoclonal antibodies to the peptides. These and other uses of the described HCV mosaic antigen composition will be apparent to those skilled in the art.

### EXAMPLE 1

### Synthesis and Characterization of HCV Peptides

The amino acid sequences of the HCV peptides listed in Table 1 were derived from the amino acid sequence of the polyprotein encoded by the HCV genome. The amino acid residues used in the peptides are given the single letter code as follows: A=Ala=alanine, R=Arg=arginine, N=Asn=asparagine, D=Asp=aspartic acid, C=Cys=cysteine, E=Glu=glutamic acid, Q=Gln=glutamine, G=Gly=glycine, H=His=histidine, I=Ile=isoleucine, L=Leu=leucine, K=Lys=lysine, M=Met=methionine, F=Phe=phenylalanine, P=Pro=proline, S=Ser=serine, T=Thr=threonine, W=Trp=tryptophan, Y=Tyr=tyrosine, and V=Val=valine.

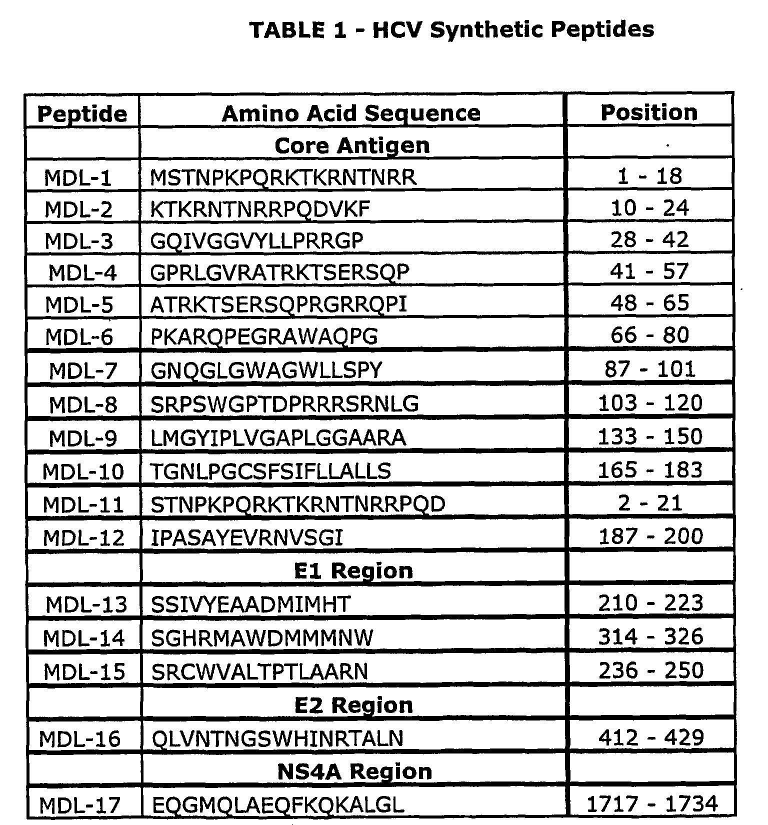

Peptides MDL-7 to MDL-17 are listed for comparison only.

The peptides of the present invention can be synthesized by standard methods for peptide synthesis such as the solid phase synthesis method.

The synthetic HCV peptides used in practicing the methods of this invention are synthesized using standard solid-phase synthesis techniques as, for example, described by Merrifield, Adv. Enzymol., 32:221-296 (1969), and Fields, G. B. and Noble, R. L., Int. J. Peptide Protein Res., 35:161-214 (1990) or for example, by the simultaneous multiple peptide synthesis method using the solid-phase technique described by Houghten, Proc. Natl. Acad. Sci. USA, 82:5131-5135 (1985). The synthesized peptides are then analyzed by reverse phase high performance liquid chromatography (HPLC) on a Vydac C-18 column (Alltech Associates, Inc., Ill.) with a 0-60% acetonitrile linear gradient in 0.1% trifluoroacetic acid. Peptides are then purified to homogeneity by preparative HPLC using optimal conditions suggested by the analytical chromatography. Amino acid compositions and concentrations of isolated peptides are determined by subjection to 24 hour hydrolysis in 6 N HCI in evacuated tubes at 110°C and subsequent analysis on a Beckman Model 6300 High Performance Analyzer.

### EXAMPLE 2

### Chemical Coupling of HCV Synthetic Peptides to a Carrier Protein

Several strategies can be employed for the preparation of the mosaic antigen composition of the present invention. Most strategies rely on the use of a panel of synthetic peptides or recombinant antigen fragments corresponding to immunodominant regions of the native antigens. These antigen components are often coupled to an immunologically inert carrier to improve immobilization and/or increase antigen density on the solid support. Both peptides and recombinant antigen fragments can be prepared for conjugation to a carrier by grafting one cysteine residue at the carboxyl terminal end or amino terminal end of the peptide or fragment thereby allowing coupling to occur via a sulfhydryl reactive cross-linker. Alternatively, the cysteine-containing peptides and recombinant antigen fragments can be directly coupled to a matrix activated with a sulfhydryl-reactive linker. In general, these linkage methods have minimal or no influence on adversely effecting antibody recognition since the antigen component is linked to the carrier component, or the solid support, via its terminal region. The molecular complex antigen/carrier can be either passively adsorbed or covalently coupled to a solid matrix (immobilized capture reagent). Several methods for both strategies are available. A general example for the preparation of the mosaic antigen composition of the present invention for anti-HCV antibody detection is provided below.

The peptides labeled MDL-1 to MDL-17 were chemically synthesized with a C-terminus cysteine residue. The peptides can be covalently coupled using a cross-linking reaction with carrier molecule such as bovine or human serum albumin, polylysine, cytochrome C, etc. Heterobifunctional cross-linker reagents SMCC [succinimidyl-4(N-maleimidomethyl)cyclohexane-1-carboxylate] and MBS [m-maleldobenzoyl-N-hydroxysuccinimide ester] are commonly used for coupling sulfhydryl-containing peptides to free amino groups of carrier proteins. The reaction is carried out in two steps. The first step consists of reacting the succinimidyl reactive group of the cross-linkers (e.g. MBS or SMCC) with the free amino groups of the carrier molecule to produce a linker-activated carrier. The second step consists of coupling the linker-activated carrier with one or more of the cysteine-containing peptides MDL-1 to MDL-17. The peptide-carrier conjugates are immobilized by passive adsorption on polystyrene microparticles, polystyrene microtiterplates, nitrocellulose membrane or other solid supports used for immunoassays.

In a preferred embodiment of the invention, the mosaic antigen composition comprising the peptides labeled MDL-1 to MDL-6 (i.e. SEQ ID NOS: 1, 2, 3, 4, 5 and 6) were chemically synthesized with a C-terminus cysteine residue (i.e. SEQ ID NOS: 7, 8, 9, 10, 11 and 12). The preferred mass ratio of peptides MDL-1, MDL-2, MDL-3, MDL-4, MDL-5 and MDL-6 to carrier used was 4:1:4:4:1:2. The peptide-carrier conjugate was immobilized by passive adsorption on a nitrocellulose membrane.

### EXAMPLE 3

### Immunofiltration Dotting Assay Format

Several versions of antibody capture assay formats are available which differ mainly according to the type of solid supports employed and the detection technology used to determine the presence of immunological complexes formed by antigen-antibody recognition and binding. A commercially available immunofiltration dotting assay procedure (MedMira Inc., Halifax, N.S.) was used to test for immunoreactivity of the peptide-carrier conjugates based on colloidal gold detection using a nitrocellulose membrane as a solid support and is described below.

The HCV positive/negative specimens used to evaluate the mosaic antigen composition were provided by Dr. Spencer Lee (Director of Virology/Immunology, Microbiology Division, Department of Pathology and Laboratory Medicine) of the Queen Elizabeth II Health Sciences Center (Halifax, Nova Scotia). All specimens were tested with the Abbott's HCV test using the Axsym system platform. The Riba-II (Chiron) immunoblot assay was used as a confirmation test.

The maleimide-activated carrier molecules (KLH, BSA and HSA) were purchased from Pierce (Rockford, IL) and conjugated with cysteine containing peptides using a peptide-carrier mass ratio of 0.5 except mentioned otherwise.

Typically, the conjugates were passively adsorbed at a concentration of 1 mg/ml (bicarbonate buffer, 50 mM, pH 9.6) by applying one microliter of peptide-carrier conjugate on the nitrocellulose membrane. The antigen dotted membrane was air-dried. Before the test, the membrane was primed with 3 drops of washing buffer (Tris/phosphate/sodium chloride, pH 9.6 containing 0.25% Tween®20). The tests were performed as follows.

One drop of specimen serum was added to the membrane and after complete absorption of the sample, the membrane was washed with 4 drops of washing buffer. Four drops of protein A/colloidal gold solution (concentration corresponding to an optical density of 2 at 520 nm) was added to the membrane and the membrane was washed with 3 more drops of washing solution. The results were recorded visually within 5 minutes after completion of the test.

### EXAMPLE 4

### Epitope Mapping and Immunoreactivity Evaluation of HCV Peptide Conjugates

The identification of major linear epitopes was carried out using selected peptides corresponding to various regions of the HCV polyprotein and panels of well-characterized HCV positive and negative human sera. Additionally, a comparison of different carrier components was made based on a sensitivity/specificity assessment of various peptide-carrier conjugates.

A series of 10 peptides covering the core (n=6), E1(n=3) and E2 (n=1) regions of the HCV polyprotein were tested for immunoreactivity using a panel of HCV positive (n=10) and HCV negative (n=10) specimens. Peptides MDL-11 and MDL-12, derived from the core region, and peptide MDL-17, derived from the NS4A region, were eventually excluded from the study due to their poor solubility in aqueous solutions.

Peptides were individually conjugated to three different carriers (BSA, HSA and KLH) and adsorbed passively on a nitrocellulose membrane support. Tests were performed by immunofiltration dotting assay format using a,commercially available flow-through assay platform (MedMira Inc., Halifax, N.S.).

Results were classified based on apparent dot intensities as follows:

| **SYMBOL** | **MEANING** |
|---|---|
| - | negative (no reaction) |
| S | shadow (borderline reaction/indeterminate) |
| W+ | weak reaction |
| +1/+2/+3/+4 | positive reaction/relative intensity (1 low → 4 high) |

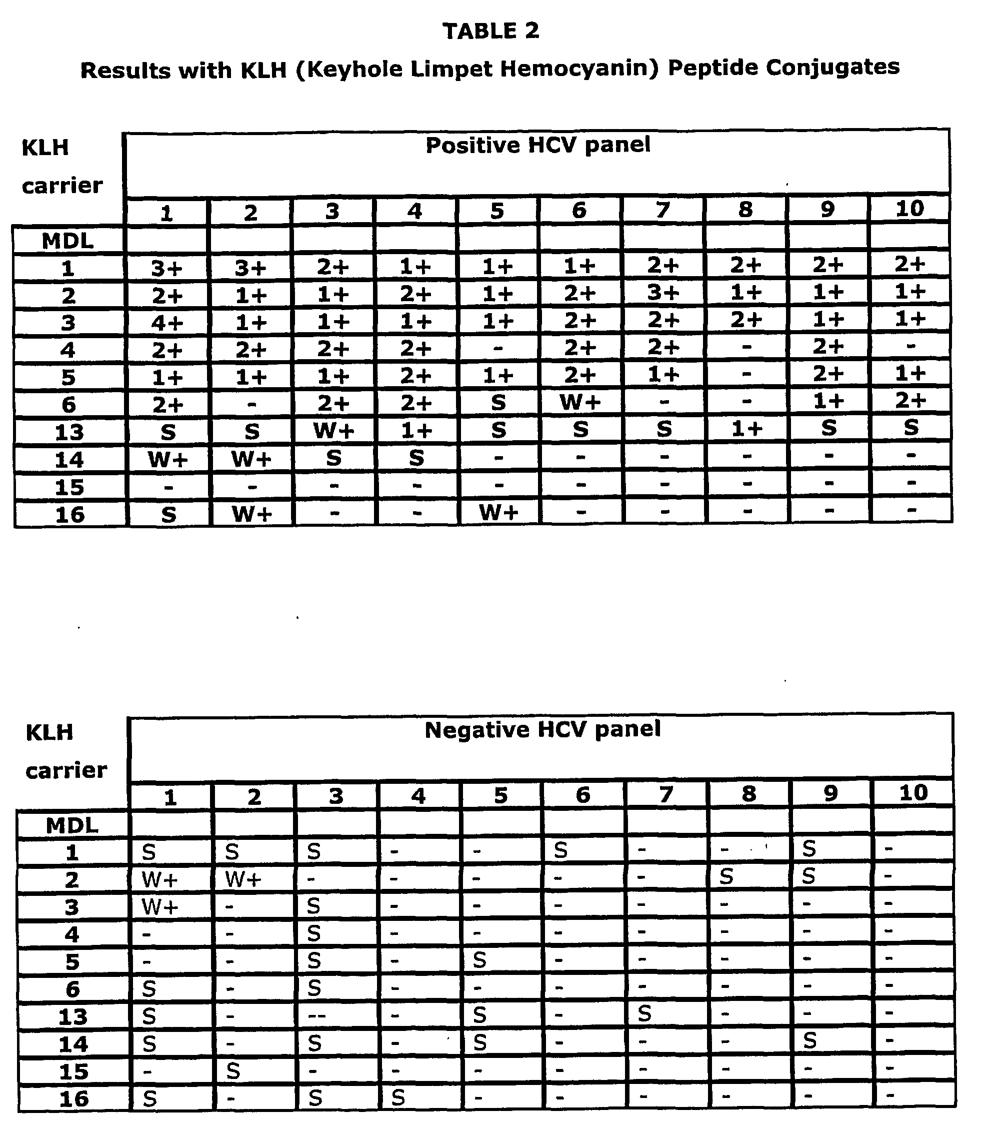

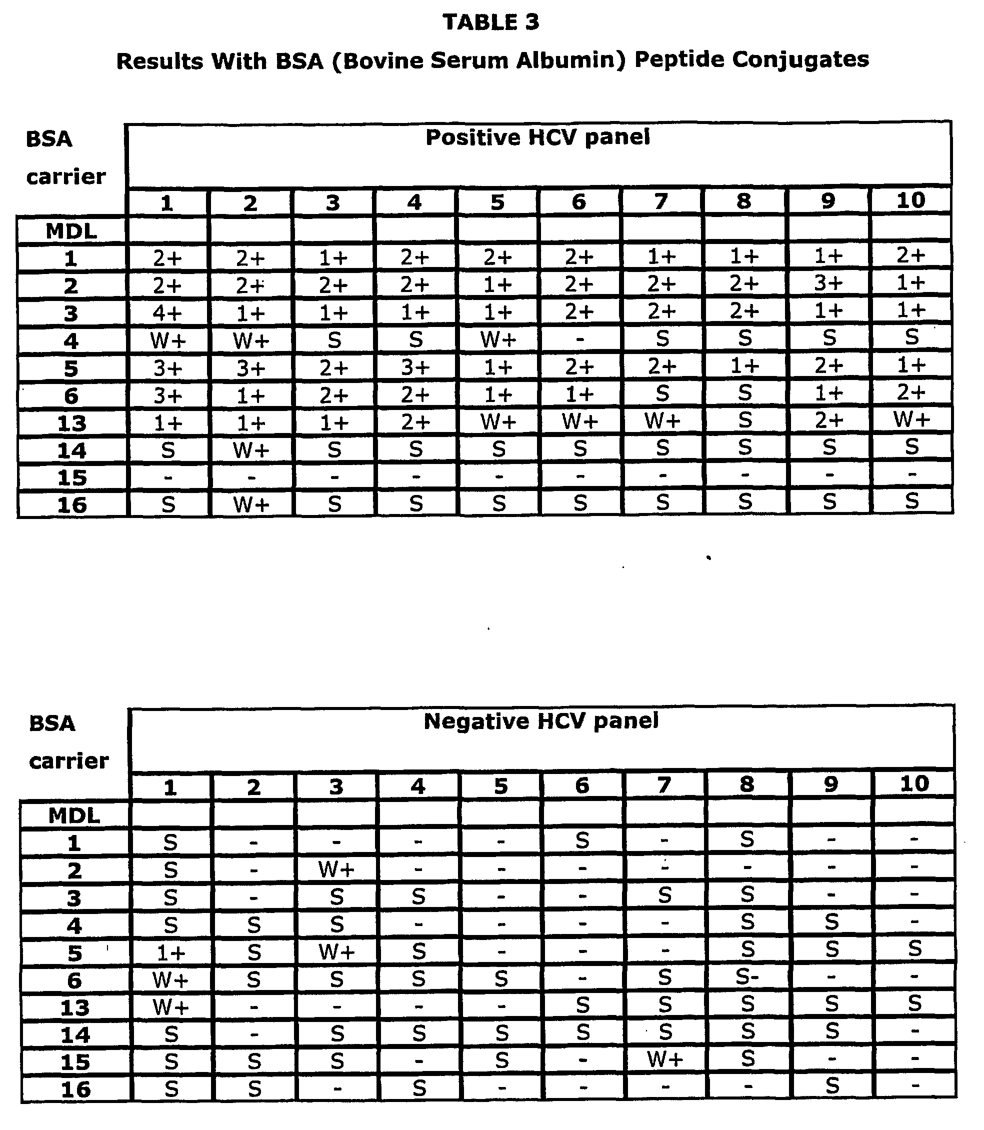

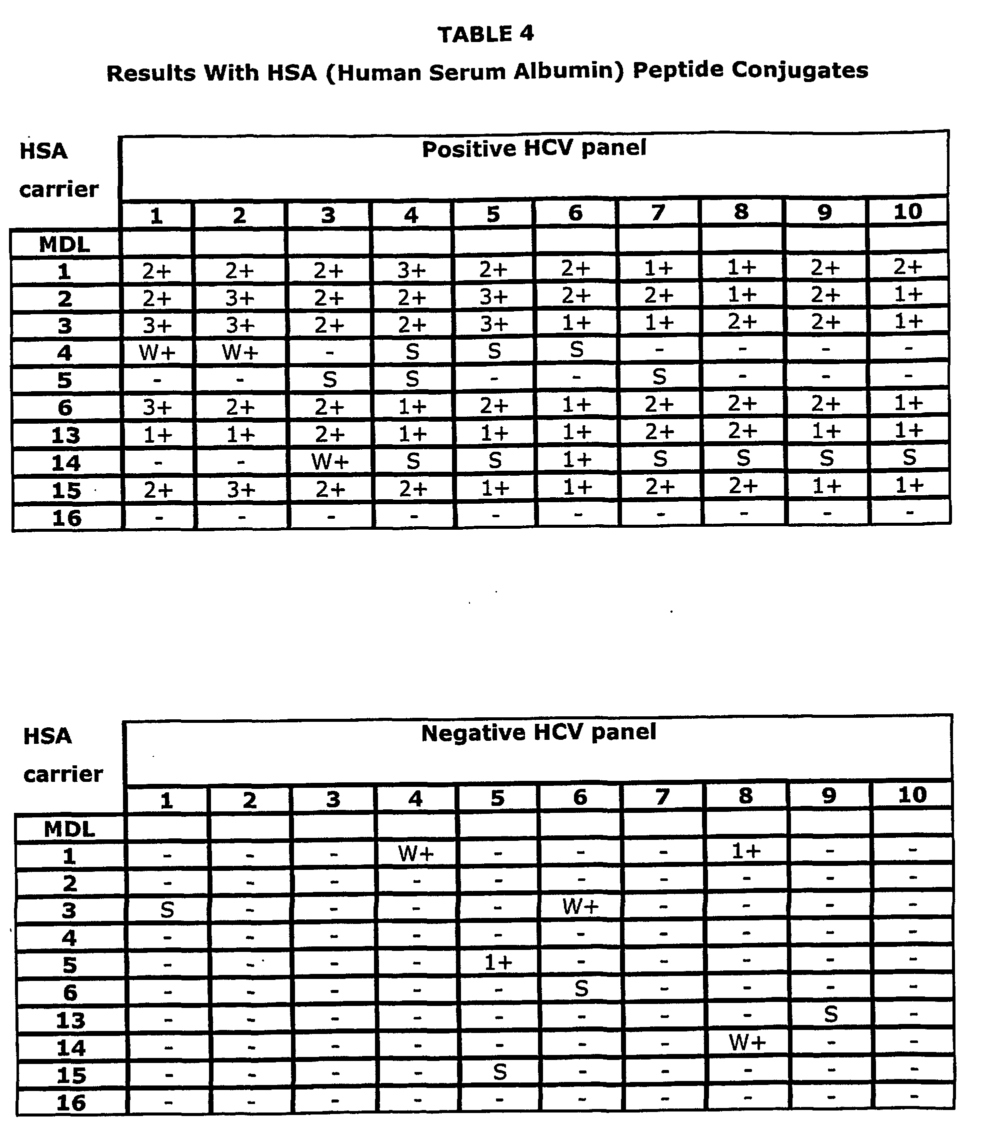

The results show that peptide conjugates prepared with HSA (followed by BSA and KLH) gave less false-positive reactions with the negative HCV samples.

Conjugates prepared with peptides MDL-1, MDL-2 and MDL-3 from the core region gave more consistent reactivity with positive HCV samples independently of the carrier used to prepare the conjugates. No significant reactivity was observed with these conjugates when tested with negative HCV samples. On the other hand, conjugates prepared with peptides MDL-4, MDL-5 and MDL-6 from the core region gave different results depending upon the type of carrier used. Peptide MDL-4 was reactive with most HCV positive samples when conjugated with KLH, but was marginally reactive or unreactive when conjugated with BSA and HSA. Peptide MDL-5 was reactive with most HCV positive samples when conjugated with KLH and BSA, but unreactive when conjugated with HSA. Peptide MDL-6 was reactive with most HCV positive samples when conjugated with HSA and BSA, but poorly reactive when conjugated with KLH.

Conjugates prepared with peptides MDL-12, MDL-13 and MDL-14 from the E1 region exhibited low and inconsistent immunoreactivity with the HCV positive samples independently of the carrier used.

Conjugates prepared with peptide MDL-15 from the E2 region were consistently unreactive or poorly reactive with the HCV positive samples independently of the carrier used.

Based on these results, HSA is a preferred carrier for HCV peptide conjugation and for application for anti-HCV antibody detection. The results suggest that HSA gives higher specificity (i.e. signal to noise ratio).

Most of the immunoreactivity is located in the core region, i.e. peptides MDL-1 to MDL-6, inclusive. The reactivity of peptides MDL-4, MDL-5 and MDL-6 appears to depend upon the type of carrier used which is likely attributed to the effect of peptide density on the carrier molecule.

Peptides derived from the E1 and the E2 regions are not suitable for HCV diagnostics.

### Example 5

### Epitope Mapping of the Core Region (Residue Positions 80 - 200)

An assessment of immunoreactivity of selected peptides corresponding to residues 80 to 200 of the core region was carried out using panels of well-characterized HCV positive and negative human sera.

A series of 4 peptides covering the core region corresponding to residues 80 to 200 were tested for immunoreactivity with panels of HCV positive (n=5) and HCV negative (n=5) specimens. Peptides were individually conjugated to HSA using a peptide to carrier mass ratio of 1 and then adsorbed passively on a nitrocellulose membrane support. Tests were performed by immunofiltration dotting assay format using a commercially available flow-through assay platform (MedMira Inc., Halifax, N.S.).

Results were classified based on apparent dot intensities as follows:

| **SYMBOL** | **MEANING** |
|---|---|
| - | negative (no reaction) |
| S | shadow (borderline reaction/indeterminate) |
| W+ | weak reaction |
| +1/+2/+3/+4 | positive reaction/relative intensity (1 low → 4 high) |

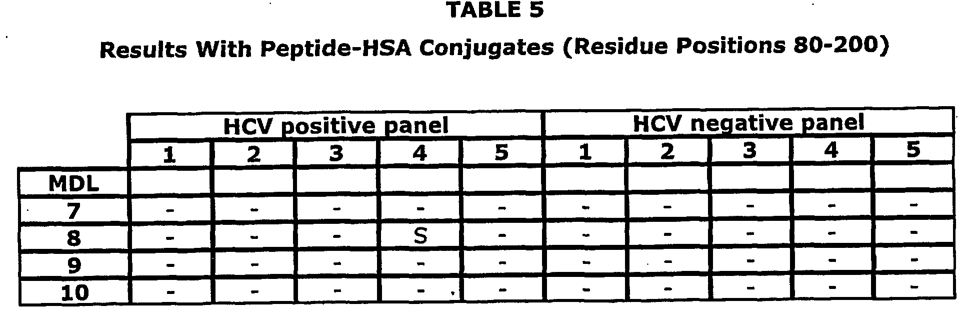

Peptides derived from the core region between residues 80 to 200 do not appear to include major immunodominant regions since none of the five HCV positive samples reacted with these peptides.

### Example 6

### Optimal Combination Evaluation of HCV Core Region Peptides

An evaluation of various combinations of peptides corresponding to residues 1 to 80 of the core region was carried out for immunoreactivity with HCV positive and negative human sera.

Various combinations of 6 peptides (i.e. MDL-1 to MDL-6) corresponding to residues 1 to 80 of the core region were tested for immunoreactivity with panels of HCV positive (n=10) and HCV negative (n=10) specimens. The peptides were individually conjugated to HSA using a peptide to carrier mass ratio of 1 and then adsorbed passively on a nitrocellulose membrane support. Tests were performed by immunofiltration dotting assay format using a commercially available flow-through assay platform (MedMira Inc., Halifax, N.S.).

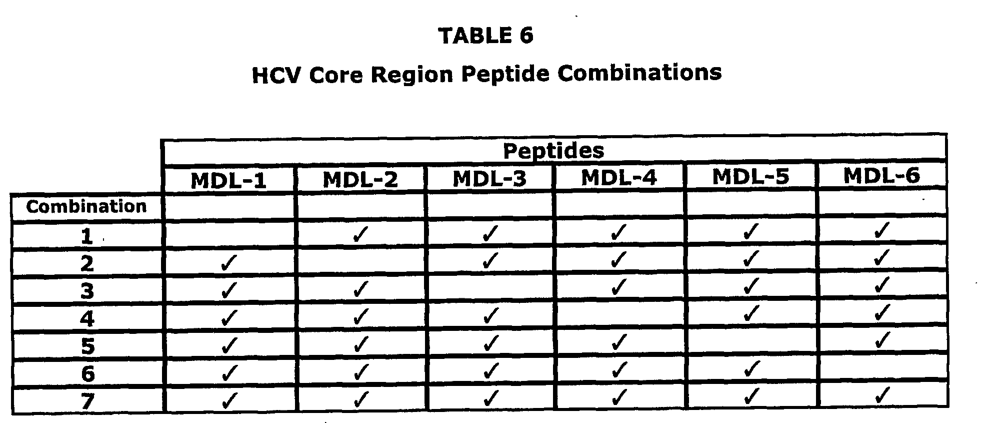

Results were classified based on apparent dot intensities as follows:

| **SYMBOL** | **MEANING** |
|---|---|
| - | negative (no reaction) |
| S | shadow (borderline reaction/indeterminate) |
| W+ | weak reaction |
| +1/+2/+3/+4 | positive reaction/relative intensity (1 low → 4 high) |

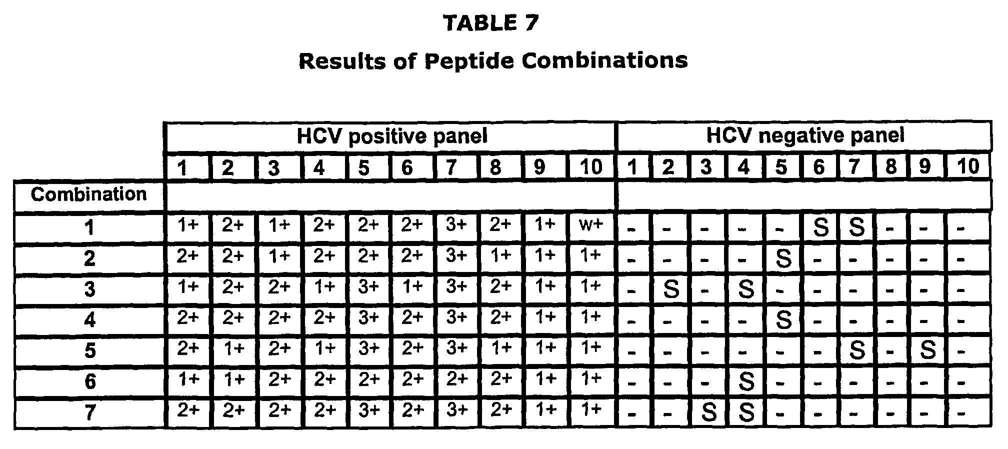

Combination 7 gives the highest sensitivity (i.e. highest dot intensity) with all 10 positive samples compared to combinations 1 to 6. All combinations give comparable specificity (i.e. no significant reaction with HCV negative specimens).

### Example 7

### Determination of Optimal Peptide to Carrier Ratio

A determination of the optimal peptide to carrier mass ratio was carried out for preparation of the mosaic antigen composition.

Peptides corresponding to residues 1 to 80 core region (peptides MDL-1 to MDL-6 inclusive) were individually conjugated to HSA at various ratios (i.e. peptide to carrier mass ratio) and adsorbed passively on a nitrocellulose membrane support. Immunoreactivity was assessed with serially diluted HCV positive serum prepared from a pool of five well-characterized HCV positive specimens. Tests were performed by immunofiltration dotting assay format using a commercially available flow-through assay platform (MedMira Inc., Halifax, N.S.). Optimal peptide to carrier ratios were determined as the ratio giving an intensity between 2+ and 1+ with the pool serum preparation diluted 1 in 5.

Results were classified based on apparent dot intensities as follows:

| **SYMBOL** | **MEANING** |
|---|---|
| - | negative (no reaction) |
| S | shadow (borderline reaction/indeterminate) |
| W+ | weak reaction |
| +1/+2 | positive reaction/relative intensity (1 low → 2 high) |

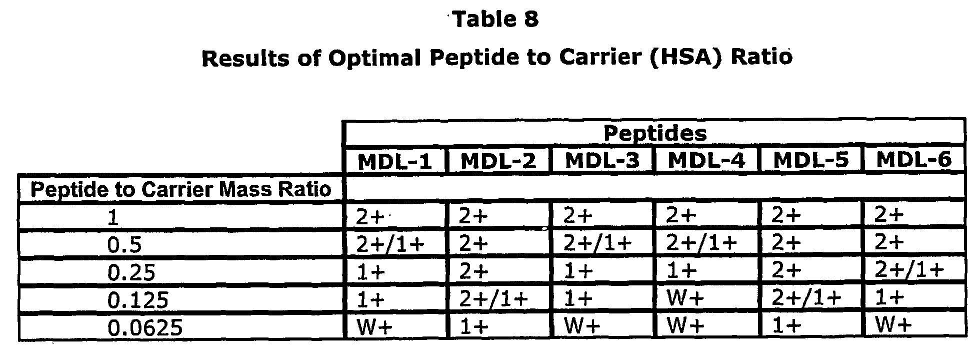

Based on the above results and a total of about 25 to 30 peptides per carrier, the optimal peptide to carrier mass ratios using peptides MDL-1, MDL-2, MDL-3, MDL-4, MDL-5 and MDL-6, and HSA as the carrier are the following:

| **MDL** | **Peptide/HSA Mass Ratio** |
|---|---|
| 1 | 0.5 |
| 2 | 0.125 |
| 3 | 0.5 |
| 4 | 0.5 |
| 5 | 0.125 |
| 6 | 0.25 |

### INDUSTRIAL APPLICABILITY

The unique combination of HCV core peptides provided as a mosaic antigen composition having the three-dimensional configuration described herein results in higher specificity and sensitivity for the detection of human antibodies specific to HCV and thus, is extremely useful in the development of reliable diagnostic methods and assays for the detection of HCV.

### SEQUENCE LISTING

(1) GENERAL INFORMATION:
   (i) APPLICANT: MedMira Inc.
   (ii) TITLE OF INVENTION: HCV Mosaic Antigen Composition
   (iii) NUMBER OF SEQUENCES: 13
   (iv) CORRESPONDENCE ADDRESS:
      (A) ADDRESSEE: G. Ronald Bell & Associates
      (B) STREET: P.O. Box 2450, Station D
      (C) CITY: Ottawa
      (D) STATE: Ontario
      (E) COUNTRY: CANADA
      (F) ZIP: K1P 5W6
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: ASCII (Text)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 2,311,022
      (B) FILING DATE: 7 July, 2000
      (C) CLASSIFICATION:
   (viii) ATTORNEY/AGENT INFORMATION:
      (A) NAME: G. Ronald Bell & Associates
      (B) REGISTRATION NUMBER:
      (C) REFERENCE/DOCKET NUMBER: 3543-001PCT
   (ix) TELECOMMUNICATION INFORMATION:
      (A) TELEPHONE: (613) 233-5684
      (B) TELEFAX: (613) 233-7941
(2) INFORMATION FOR SEQ ID NO:1:
   (i) SEQUENCE CHARACTERISTICS:
      (A) LENGTH: 18 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Lys Thr Lys Arg Asn Thr Asn Arg Arg Pro Gln Asp Val Lys Phe
   1 5 10 15
(2) INFORMATION FOR SEQ ID NO:3:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Gly Gln Ile Val Gly Gly Val Tyr Leu Leu Pro Arg Arg Gly Pro
   1 5 10 15
(2) INFORMATION FOR SEQ ID NO:4:
   (i) SEQUENCE CHARACTERISTICS:
      (A) LENGTH: 18 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
(2) INFORMATION FOR SEQ ID NO:5:
   (i) SEQUENCE CHARACTERISTICS:
      (A) LENGTH: 18 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:6:
   Pro Lys Ala Arg Gln Pro Glu Gly Arg Ala Trp Ala Gln Pro Gly
   1 5 10 15
(2) INFORMATION FOR SEQ ID NO:7:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:7:
(2) INFORMATION FOR SEQ TD NO:8:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Gly Gln Ile Val Gly Gly Val Tyr Leu Leu Pro Arg Arg Gly Pro Cys
   1 5 10 15
(2) INFORMATION FOR SEQ ID NO:10:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:10:
(2) INFORMATION FOR SEQ ID NO:11:
   (i) SEQUENCE CHARACTERISTICS:
      (A) LENGTH: 19 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:11:
   (2) INFORMATION FOR SEQ ID NO:12:
      (i) SEQUENCE CHARACTERISTICS:
         (A) LENGTH: 16 amino acids
         (B) TYPE: amino acid
         (C) STRANDEDNESS: single
         (D) TOPOLOGY: linear
      (ii) MOLECULE TYPE: peptide
      (xi) SEQUENCE DESCRIPTION: SEQ ID NO:12:
   Pro Lys Ala Arg Gln Pro Glu Gly Arg Ala Trp Ala Gln Pro Gly Cys
   1 5 10 15
   (2) INFORMATION FOR SEQ ID NO:13:
      (i) SEQUENCE CHARACTERISTICS:
         (A) LENGTH: 120 amino acids
         (B) TYPE: amino acid
         (C) STRANDEDNESS: single
         (D) TOPOLOGY: linear
      (ii) MOLECULE TYPE: peptide
      (xi) SEQUENCE DESCRIPTION: SEQ ID NO:13:
   Met Ser Thr Asn Pro Lys Pro Gln Lys Lys Asn Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Gln Pro Glu Gly Arg Ala Trp Ala Gln Pro Gly
   65 70 75 80
   Tyr Pro Trp Pro Leu Tyr Gly Asn Glu Gly Cys Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly
   115 120

## Claims

1. A peptide composition comprising two or more different peptides that are recognized by anti-HCV antibodies, **characterized in that** each peptide:
consists of an amino acid sequence selected from the group consisting of MSTNPKPQRKTKRNTNRR (SEQ ID NO:1), KTKRNTNRRPQDVKF (SEQ ID NO:2), GQIVGGVYLLPRRGP (SEQ ID NO:3), GPRLGVRATRKTSERSQP (SEQ ID NO:4), ATRKTSERSQPRGRRQPI (SEQ ID NO:5), and PKARQPEGRAWAQPG (SEQ ID NO:6);
is covalently coupled N-terminally or C-terminally to a carrier component; and
has a peptide to carrier mass ratio in the range of 0.5 to 8.0.

2. The peptide composition according to claim 1 **characterized** as comprising the peptides MSTNPKPQRKTKRNTNRR (SEQ ID NO:1), KTKRNTNRRPQDVKF (SEQ ID NO:2), GQIVGGVYLLPRRGP (SEQ ID NO:3), GPRLGVRATRKTSERSQP (SEQ ID NO:4), ATRKTSERSQPRGRRQPI (SEQ ID NO:5), and PKARQPEGRAWAQPG (SEQ ID NO:6).

3. The peptide composition according to claim 1 or 2, **characterized in that** for each peptide, the peptide to carrier mass ratio is in the range of 1.0 to 4.0.

4. The peptide composition according to claim 2, **characterized in that** the peptide to carrier mass ratios for the peptides MSTNPKPQRKTKRNTNRR (SEQ ID NO:1), KTKRNTNRRPQDVKF (SEQ ID NO:2), GQIVGGVYLLPRRGP (SEQ ID NO:3), GPRLGVRATRKTSERSQP (SEQ ID NO:4), ATRKTSERSQPRGRRQPI (SEQ ID NO:5), and PKARQPEGRAWAQPG (SEQ ID NO:6) are 4, 1, 4, 4, 1 and 2, respectively.

5. The peptide composition according to any one of claims 1 to 4 **characterized in that** each peptide comprises an additional C-terminal cysteine residue.

6. The peptide composition according to claim 5, **characterized in that** said carrier component is a protein.

7. The peptide composition according to claim 6, **characterized in that** said protein is human serum albumin (HSA).

8. The peptide composition according to claim 6 or 7, **characterized in that** each peptide is covalently coupled to said protein via the C-terminal cysteine residue using a cross-linking agent.

9. The peptide composition according to claim 8, **characterized in that** said cross-linking agent is selected from the group consisting of MBS (*m*-maleimidobenzoyl-*N*-hydroxysuccinimide ester), sulfo-MBS (*m*-maleimidobenzoyl-*N*-hydroxysulfosuccinimide ester), SIAB (*N*-succinimidyl-4-(iodoacetyl)aminobenzoate), sulfo-SIAB (sulfosuccinimidyl-4-(iodoacetyl)aminobenzoate, SMCC (succinimidyl-4-(*N*-maleimidomethyl)-cyclohexane-1-carboxylate), sulfo-SMCC (sulfosuccinimidyl-4-(*N*-maleimidomethyl)cyclohexane-1-carboxylate), SMPB (succinimidyl-4-(*p*-maleimidophenyl)-butyrate), sulfo-SMPB (sulfosuccinimidyl-4-(*p*-maleimidophenyl)butyrate), LC-SPDP (succinimidyl-6-[3-(2-pyridyldithio)-propionate]hexanoate), sulfo-LC-SPDP (sulfosuccinimidyl-6-[3-(2-pyridyldithio)-propionate]hexanoate), GMBS (N-(γ-maleimidobutyryloxy)succinimide ester), sulfo-GMBS (N-(γ-maleimidobutyryloxy)sulfosuccinimide ester), SMPT (4-succinimidyl-oxycarbonyl-α-methyl-α-(2-pyridyldithio)toluene), sulfo-LC-SMPT (sutfosuccinimidyl-6-[α-methyl-α-(2-pyridyldithio)toluamido]hexanoate).

10. The peptide composition according to claim 8, **characterized in that** said cross-linking agent is *m*-maleimidobenzoyl-N-hydroxysuccinimide ester (MBS).

11. An in vitro method for the detection of antibodies to hepatitis C virus (HCV) in a subject, **characterized** as comprising the steps of:
contacting a biological test sample from said subject with a peptide composition according to any one of claims 1 to 10; and
detecting an immune complex formed between said peptide composition and the antibodies to HCV present in said biological test sample; wherein
the presence of said immune complex is indicative of the presence of antibodies to HCV in said subject.

12. The in vitro method according to claim 11, **characterized in that** said peptide composition is immobilized to a solid support.

13. The in vitro method according to claim 12, **characterized in that** said solid support is a nitrocellulose membrane.

14. The in vitro method according any one of claims 11 to 13, **characterized in that** the presence of said immune complex is determined by the addition of an indicator reagent.

15. The in vitro method according to claim 14, **characterized in that** said indicator reagent comprises a signal-generating component attached to a molecule capable of binding specifically to a human anti-HCV antibody.

16. The in vitro method according to claim 15, **characterized in that** said signal-generating component is colloidal gold.

17. The in vitro method according to claim 15 or 16, **characterized in that** said molecule capable of binding specifically to a human anti-HCV antibody is a mammalian anti-human immunoglobulin or staphylococcal protein A.

18. A kit for detecting antibodies to hepatitis C virus (HCV) in a subject **characterized** as comprising:
a peptide composition according to any one of claims 1 to 10 in a suitable container, wherein said peptide composition is immobilized to a solid support; and
an indicator reagent.

19. The kit according to claim 18, further comprising:
- control standards; and
- a specimen diluent or washing buffer.

20. The kit according to claim 18 or 19, **characterized in that** said solid support is a nitrocellulose membrane.

21. The kit according to any one of claims 18 to 20, **characterized in that** said indicator reagent comprises a signal-generating component attached to a molecule capable of binding specifically to a human anti-HCV antibody.

22. The kit according to claim 21, **characterized in that** the signal-generating component is colloidal gold.

23. The kit according to claim 21 or 22 **characterized in that** said molecule capable of binding specifically to a human anti-HCV antibody is a mammalian anti-human immunoglobulin or staphylococcal protein A.

24. The kit according to any one of claims 18 to 23 **characterized in that** it is provided in an immunofiltration dotting assay format.

## Patentansprüche

1. Eine aus zwei oder mehreren verschiedenen Peptiden bestehende Peptidverbindung, die von Anti-HCV-Antikörpern erkannt wird, **dadurch gekennzeichnet, daß** jedes Peptid:
aus einer Aminosäure-Sequenz besteht, die aus der folgenden Gruppe ausgewählt worden ist: MSTNPKPQRKTKRNTNRR (SEQ ID NR: 1), KTKRNTNRRPQDVKF (SEQ ID NR: 2), GQIVGGVYLLPRRGP (SEQ ID NR: 3), GPRLGVRATRKTSERSQP (SEQ ID NR: 4), ATRKTSERSQPRGRRQPI (SEQ ID NR: 5) und PKARQPEGRAWAQPG (SEQ ID NR: 6);
kovalent mit ihrer N-Endgruppe oder C-Endgruppe an eine Trägerkomponente gekoppelt ist,
und
ein Peptid-zu-Träger-Massenverhältnis von 0,5 bis 8,0 besitzt.

2. Peptidverbindung gemäß Anspruch 1, **dadurch gekennzeichnet, daß** sie aus den Peptiden MSTNPKPQRKTKRNTNRR (SEQ ID NR: 1), KTKRNTNRRPQDVKF (SEQ ID NR: 2), GQIVGGVYLLPRRGP (SEQ ID NR: 3), GPRLGVRATRKTSERSQP (SEQ ID NR: 4), ATRKTSERSQPRGRRQPI (SEQ ID NR: 5) und PKARQPEGRAWAQPG (SEQ ID NR: 6) besteht.

3. Peptidverbindung gemäß Anspruch 1 oder 2, **dadurch gekennzeichnet, daß** für jedes Peptid das Peptid-zu-Träger-Massenverhältnis 1,0 bis 4,0 ist.

4. Peptidverbindung gemäß Anspruch 2, **dadurch gekennzeichnet, daß** das Peptid-zu-Träger-Massenverhältnis für die Peptide MSTNPKPQRKTKRNTNRR (SEQ ID NR: 1), KTKRNTNRRPQDVKF (SEQ ID NR: 2), GQIVGGVYLLPRRGP (SEQ ID NR: 3), GPRLGVRATRKTSERSQP (SEQ ID NR: 4), ATRKTSERSQPRGRRQPI (SEQ ID NR: 5) und PKARQPEGRAWAQPG (SEQ ID NR: 6) jeweils 4, 1, 4, 4, 1 und 2 ist.

5. Peptidverbindung gemäß Ansprüch 1, 2, 3 oder 4, **dadurch gekennzeichnet, daß** jedes Peptid einen zusätzlichen C-Endgruppe-Cysteinrest enthält.

6. Peptidverbindung gemäß Anspruch 5, **dadurch gekennzeichnet, daß** die Trägerkomponente ein Protein ist.

7. Peptidverbindung gemäß Anspruch 6, **dadurch gekennzeichnet, daß** das Protein das Humanserumalbumin (HSA) ist.

8. Peptidverbindung gemäß Anspruch 6 oder 7, **dadurch gekennzeichnet, daß** jedes Peptid kovalent mit dem C-Endgruppe-Cysteinrest mit Hilfe eines Vernetzungsmittels an das Protein gekoppelt ist.

9. Peptidverbindung gemäß Anspruch 8, **dadurch gekennzeichnet, daß** das Vernetzungsmittel aus einer Gruppe ausgewählt ist, die aus MBS (m-Maleimido-Benzoyl-N-Hydroxy-Succinimidester), Sulfo-MBS (m-Maleimido-Benzoyl-N-Hydroxy-Sulfosuccinimidester), SIAB (N-Succinimidyl-4-(Iodacetyl)-Aminobenzoat), Sulfo-SIAB (Sulfosuccinimidyl-4-(Iodacetyl)-Aminobenzoat), SMCC (Succinimidyl-4-(N-Maleimidomethyl)-Cyclohexan-1-Carboxylat), Sulfo-SMCC (Sulfosuccinimidyl-4-(N-Maleimidomethyl)-Cyclohexan-1-Carboxylat), SMPB (Succinimidyl-4-(p-Maleimidophenyl)-Butyrat), Sulfo-SMPB (Sulfosuccinimidyl-4-(p-Maleimidophenyl)-Butyrat), LC-SPDP (Succinimidyl-6-[3-(2-Pyridyldithio)-Propionat]-Hexanoat), Sulfo-LC-SPDP (Sulfosuccinimidyl-6-[3-(2-Pyridyldithio)-Propionat]-Hexanoat), GMBS (N-(γ-Maleimidobutyryloxy)-Succinimidester), Sulfo-GMBS (N-(γ-Maleimidobutyryloxy)-Sulfosuccinimidester), SMPT (4-Succinimidyl-Oxycarbonyl-α-Methyl-α-(2-Pyridyldithio)-Toluen), Sulfo-LC-SMPT (Sulfosuccinimidyl-6-[α-Methyl-α-(2-Pyridyldithio)-Toluamid]-Hexanoat) besteht.

10. Peptidverbindung gemäß Anspruch 8, **dadurch gekennzeichnet, daß** das Vernetzungsmittel m-Maleimido-Benzoyl-N-Hydroxy-Succinimidester (MBS) ist.

11. In-vitro-Verfahren zur Ermittlung von Antikörpern zum Hepatitis-C-Virus (HCV) in einem Patienten, **gekennzeichnet durch** die folgenden Schritte:
das Kontaktieren eines biologischen Prüfkörpers aus dem Patienten mit einer Peptidverbindung gemäß einem der Ansprüche 1 bis 10; und
die Ermittlung eines zwischen der Peptidverbindung und den in dem biologischen Prüfkörper anwesenden Antikörpern zu HCV gebildeten Immunkomplexes, wobei die Anwesenheit des Immunkomplexes auf die Anwesenheit von Antikörpern zu HCV in dem Patienten schliessen läßt.

12. In-vitro-Verfahren gemäß Anspruch 11, **dadurch gekennzeichnet, daß** die Peptidverbindung zu einer festen Stütze immobilisiert wird.

13. In-vitro-Verfahren gemäß Anspruch 12, **dadurch gekennzeichnet, daß** die feste Stütze eine Nitrocellulosen-Membrane ist.

14. In-vitro-Verfahren gemäß Anspruch 11, 12 oder 13, **dadurch gekennzeichnet, daß** die Anwesenheit des erwähnten Immunkomplexes durch den Zusatz eines Indikatorreagenses ermittelt wird.

15. In-vitro-Verfahren gemäß Anspruch 14, **dadurch gekennzeichnet, daß** das Indikatorreagens eine signalerzeugende Komponente enthält, die an ein Molekül gekoppelt ist, das imstande ist, sich spezifisch an einen Human-Anti-HCV-Antikörper zu koppeln.

16. In-vitro-Verfahren gemäß Anspruch 15, **dadurch gekennzeichnet, daß** die signalerzeugende Komponente Kolloidgold ist.

17. In-vitro-Verfahren gemäß Anspruch 15 oder 16, **dadurch gekennzeichnet, daß** das Molekül, das imstande ist, sich spezifisch an einen Human-Anti-HCV-Antikörper zu koppeln, ein Mammalien-Anti-Human-Immunoglobulin oder ein Staphylokokken-Protein A ist.

18. Prüfsatz zur Ermittlung von Antikörpern zu Hepatitis-C-Virus (HCV) in einem Patienten, **dadurch gekennzeichnet, daß** es die folgenden Komponenten enthält:
eine Peptidverbindung gemäß einem der Ansprüche 1 bis 10 in einem geeigneten Behälter, wobei die Peptidverbindung zu einer festen Stütze immobilisiert ist; und
ein Indikatorreagens.

19. Prüfsatz gemäß Anspruch 18, der ferner folgendes enthält:
- Kontrollnormen; und
- ein Prüfkörper-Verdünnungsmittel oder einen Waschpuffer.

20. Prüfsatz gemäß Anspruch 18 oder 19, **dadurch gekennzeichnet, daß** die feste Stütze eine Nitrocellulosen-Membrane ist.

21. Prüfsatz gemäß Anspruch 18, 19 oder 20, **dadurch gekennzeichnet, daß** das Indikatorreagens eine signalerzeugende Komponente enthält, die an ein Molekül gekoppelt ist, das imstande ist, sich spezifisch an einen Human-Anti-HCV-Antikörper zu koppeln.

22. Prüfsatz gemäß Anspruch 21, **dadurch gekennzeichnet, daß** die signalerzeugende Komponente Kolloidgold ist.

23. Prüfsatz gemäß Anspruch 21 oder 22, **dadurch gekennzeichnet, daß** das Molekül, das imstande ist, sich spezifisch an einen Human-Anti-HCV-Antikörper zu koppeln, ein Mammalien-Anti-Human-Immunoglobulin oder ein Staphylokokken-Protein A ist.

24. Prüfsatz gemäß Anspruch 18, 19, 20, 21, 22 oder 23, **dadurch gekennzeichnet, daß** er in einer eine Punktprüfung der Immunfiltration erlaubenden Form ausgeführt ist.

## Revendications

1. Composition peptidique comprenant deux peptides différents ou plus qui sont reconnus par des anticorps anti-VHC, **caractérisée en ce que** chaque peptide :
consiste en une séquence d'aminoacides sélectionnée du groupe consistant en MSTNPKPQRKTKRNTNRR ( N° D=ID DE SÉQ : 1), KTKRNTNRRPQDVKF (N° D=ID DE SÉQ : 2), GQIVGGVYLLPRRGP (N° D=ID DE SÉQ : 3), GPRLGVRATRKTSERSQP (N° D=ID DE SÉQ : 4), ATRKTSERSQPRGRRQPI (N° D=ID DE SÉQ : 5), et PKARQPEGRAWAQPG (N° D=ID DE SÉQ : 6);
est couplée par covalence à l=extrémité N ou à l=extrémité C à une composante porteuse;
et
a un ratio de peptide à la masse porteuse dans l=intervalle de 0,5 à 8,0.

2. Composition peptidique selon la revendication 1, **caractérisée en ce** qu=elle comprend les peptides MSTNPKPQRKTKRNTNRR ( N° D=ID DE SÉQ : 1), KTKRNTNRRPQDVKF (N° D=ID DE SÉQ : 2), GQIVGGVYLLPRRGP (N° D=ID DE SÉQ : 3), GPRLGVRATRKTSERSQP (N° D=ID DE SÉQ : 4), ATRKTSERSQPRGRRQPI (N° D=ID DE SÉQ : 5), et PKARQPEGRAWAQPG (N° D=ID DE SÉQ : 6).

3. Composition peptidique selon la revendication 1 ou 2, **caractérisée en ce que** pour chaque peptide, le ratio de peptide à la masse porteuse est dans l=intervalle de 1,0 à 4, 0.

4. Composition peptidique selon la revendication 2, **caractérisée en ce que** les ratios de peptide à la masse porteuse pour les peptides MSTNPKPQRKTKRNTNRR ( N° D=ID DE SÉQ : 1), KTKRNTNRRPQDVKF (N° D=ID DE SÉQ : 2), GQIVGGVYLLPRRGP (N° D=ID DE SÉQ : 3), GPRLGVRATRKTSERSQP (N° D=ID DE SÉQ : 4), ATRKTSERSQPRGRRQPI (N° D=ID DE SÉQ : 5), et PKARQPEGRAWAQPG (N° D=ID DE SÉQ : 6) sont de 4, 1, 4, 4, 1 et 2, respectivement.

5. Composition peptidique selon l=une des revendications 1 à 4, **caractérisée en ce que** chaque peptide comprend un résidu de cystéine supplémentaire à l=extrémité C.

6. Composition peptidique selon la revendication 5, **caractérisée en ce que** ladite composante porteuse est une protéine.

7. Composition peptidique selon la revendication 6, **caractérisée en ce que** ladite protéine est de l=albumine sérique humaine (ASH).

8. Composition peptidique selon la revendication 6 ou 7, **caractérisée en ce que** chaque peptide est couplé par covalence à ladite protéine en passant par le résidu de cystéine à l=extrémité C au moyen d=un agent de réticulation.

9. Composition peptidique selon la revendication 8, **caractérisée en ce que** ledit agent de réticulation est sélectionné du groupe consistant en MBS (*m*-maléimidobenzoyle-*N*-hydroxysuccinimide ester), sulfo-MBS (*m*-maléimidobenzoyle-*N*-hydroxysulfosuccinimide ester), SIAB (*N*-succidinimidyle-4-(iodoacétyle)aminobenzoate), sulfo-SIAB (sulfosuccinimidyle-4-(iodoacétyle)aminobenzoate, SMCC (succinimidyle-4-(*N*-maléimidomethyle)-cyclohexane-1-carboxylate), sulfo-SMCC (sulfosuccinimidyle-4-(*N*-maléimidomethyle)-cyclohexane-1-carboxylate), SMPB (succinimidyle-4-(*p*-maléimidophényle)-butyrate), sulfo-SMPB (sulfosuccinimidyle-4-(*p*-maléimidophenyle)-butyrate), LC-SPDP (succinimidyle-6-[3-(2-pyridyldithio)-propionate]hexanoate), sulfo-LC-SPDP (sulfosuccinimidyle-6-[3-(2-pyridyldithio)-propionate]hexanoate), GMBS (N-(γ-maléimidobutyryloxy)succinimide ester), sulfo-GMBS (N-(γ-maleimidobutyryloxy)sulfosuccinimide ester), SMPT (4-succinimidyle-oxycarbonyle-α-méthyle-α-(2-pyridyldithio)toluène), sulfo-LC-SMPT (sulfosuccinimidyle-6-[α-méthyle-α-(2-pyridyldithio)toluamido]hexanoate).

10. Composition peptidique selon la revendication 8, **caractérisée en ce que** ledit agent de réticulation est le m-maléimidobenzoyle-*N*-hydroxysuccinimide ester (MBS).

11. Méthode in vitro pour la détection des anticorps au virus de l=hépatite C (VHC) chez un sujet, **caractérisée en ce** qu=elle comprend les étapes suivantes :
prélever un échantillon biologique pour essai dudit sujet ayant une composition peptidique selon l=une des revendications 1 à 10; et
déceler un complexe immun formé entre ladite composition peptidique et les anticorps au VHC présents dans ledit échantillon biologique pour essai; dans lequel la présence dudit complexe immun indique la présence d=anticorps au VHC chez ledit sujet.

12. Méthode in vitro selon la revendication 11, **caractérisée en ce que** ladite composition peptidique est immobilisée sur un support solide.

13. Méthode in vitro selon la revendication 12, **caractérisée en ce que** ledit support solide est une membrane en nitrocellulose.

14. Méthode in vitro selon l=une des revendications 11 à 13, **caractérisée en ce que** la présence dudit complexe immun est déterminé par l=ajout d=un indicateur réactif.

15. Méthode in vitro selon la revendication 14, **caractérisée en ce que** ledit indicateur réactif comprend une composante émettrice de signal attachée à une molécule capable de se lier spécifiquement à un anticorps humain anti-VHC.

16. Méthode in vitro selon la revendication 15, **caractérisée en ce que** ladite composante émettrice de signal est de l=or colloïdal.

17. Méthode in vitro selon la revendication 15 ou 16, **caractérisée en ce que** ladite molécule capable de se lier spécifiquement à un anticorps humain anti-VHC est une immunoglobuline anti-humaine mammalienne ou une protéine A staphylococcique.

18. Trousse pour déceler les anticorps au virus de l=hépatite C (VHC) chez un sujet, **caractérisée en ce** qu=elle comprend :
une composition peptidique selon l=une des revendications 1 à 10 dans un conteneur approprié, dans lequel la composition peptidique est immobilisée sur un support solide ; et
un réactif indicateur.

19. Trousse selon la revendication 18 qui comprend aussi :
- des normes de contrôle; et
- un diluant de spécimen ou une solution tampon de lavage.

20. Trousse selon la revendication 18 ou 19, **caractérisée en ce que** ledit support solide est une membrane en nitrocellulose.

21. Trousse selon l=une des revendications 18 à 20, **caractérisée en ce que** le réactif indicateur comprend une composante émettrice de signal attachée à une molécule capable de se lier spécifiquement à un anticorps humain anti-VHC.

22. Trousse selon la revendication 21, **caractérisée en ce que** la composante émettrice de signal est de l=or colloïdal.

23. Trousse selon la revendication 21 ou 22, **caractérisée en ce que** ladite molécule capable de se lier spécifiquement à un anticorps humain anti-VHC est une immunoglobuline anti-humaine mammalienne ou une protéine A staphylococcique.

24. Trousse selon l=une des revendications 18 à 23, **caractérisée en ce** qu=elle est fournie sous forme d=analyse par immunofiltration.
